(19) **Europäisches Patentamt / European Patent Office / Office européen des brevets**

(11) **EP 4 426 303 B1**

(12) # EUROPEAN PATENT SPECIFICATION

(45) Date of publication and mention
of the grant of the patent:
**08.10.2025  Bulletin 2025/41**

(21) Application number: **22818475.0**

(22) Date of filing: **02.11.2022**

(51) International Patent Classification (IPC):
***A61K 31/4709*** (2006.01)   ***A61K 31/519*** (2006.01)
***A61P 35/00*** (2006.01)

(52) Cooperative Patent Classification (CPC):
(C-Sets available)
**A61K 31/519; A61K 31/4709; A61P 35/00**   (Cont.)

(86) International application number:
**PCT/HU2022/050077**

(87) International publication number:
**WO 2023/079320 (11.05.2023 Gazette 2023/19)**

(54) **FARNESYL-TRANSFERASE INHIBITORS AND KRAS INHIBITORS FOR TREATING KRAS MUTANT CANCERS**

FARNESYL-TRANSFERASE INHIBITOREN UND KRAS INHIBITOREN ZUR BEHANDLUNG VON KRAS MUTANT KREBS

INHIBITEURS DE FARNESYL-TRANSFERASE ET INHIBITEURS DU KRAS POUR TRAITER LES CANCERS AVEC DES MUTATIONS KRAS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:  **02.11.2021  HU 2100375**

(43) Date of publication of application:
**11.09.2024  Bulletin 2024/37**

(60) Divisional application:
**25195450.9**

(73) Proprietors:
- **Semmelweis Egyetem**
  **1085 Budapest (HU)**
- **Kineto Lab Kft.**
  **1032 Budapest (HU)**
- **Eötvös Loránd Tudományegyetem**
  **1053 Budapest (HU)**
- **HUN-REN TERMÉSZETTUDOMÁNYI KUTATÓKÖZPONT**
  **1117 Budapest (HU)**

(72) Inventors:
- **TÍMÁR, József**
  **1112 Budapest (HU)**

- **HEGEDÜS, Balázs**
  **1118 Budapest (HU)**
- **BARANYI, Marcell**
  **1136 Budapest (HU)**
- **MOLNÁR, Eszter**
  **2040 Budaörs (HU)**
- **TÓVÁRI, József**
  **2096 Budapest (HU)**
- **RANDELOVIC, Ivan**
  **Jagodina 35000 (RS)**
- **PERCZEL, András**
  **1053 Budapest (HU)**
- **KESERÜ, György**
  **1117 Budapest (HU)**
- **BUDAY, László**
  **1117 Budapest (HU)**

(74) Representative: **Danubia Patent & Law Office LLC**
**Bajcsy-Zsilinszky út 16**
**1051 Budapest (HU)**

(56) References cited:
**WO-A1-2017/031101     WO-A1-2022/125971**
**WO-A2-2020/106647**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- NAGASAKA MISAKO ET AL: "KRAS Inhibitors-yes but what next? Direct targeting of KRAS-vaccines, adoptive T cell therapy and beyond", CANCER TREATMENT REVIEWS, ELSEVIER, AMSTERDAM, NL, vol. 101, 21 October 2021 (2021-10-21), XP086859462, ISSN: 0305-7372, [retrieved on 20211021], DOI: 10.1016/J.CTRV.2021.102309
- BANNOURA SAHAR F ET AL: "Targeting KRAS in pancreatic cancer: new drugs on the horizon", CANCER METASTASIS, KLUWER ACADEMIC PUBLISHERS, DORDRECHT, NL, vol. 40, no. 3, 1 September 2021 (2021-09-01), pages 819 - 835, XP037603794, ISSN: 0167-7659, [retrieved on 20210909], DOI: 10.1007/S10555-021-09990-2
- DELAHAYE C ET AL: "Using Tipifarnib to prevent resistance to targeted therapies in oncogene-addicted tumors", EUROPEAN JOURNAL OF CANCER, ELSEVIER, AMSTERDAM NL, vol. 174, 1 October 2022 (2022-10-01), XP087220466, ISSN: 0959-8049, [retrieved on 20221028], DOI: 10.1016/S0959-8049(22)00948-0

(52) Cooperative Patent Classification (CPC): (Cont.)

C-Sets
A61K 31/4709, A61K 2300/00;
A61K 31/519, A61K 2300/00

**Description**

FIELD OF THE INVENTION

The invention relates to cancer biology, more specifically to the treatment of KRAS mutant cancers. A potent cancer therapy is provided by the combination of a farnesyl transferase inhibitor (FTI) compound and a KRAS-inhibitor compound.

BACKGROUND OF THE INVENTION

The RAS proteins are members of the small GTPase protein family and serve as a binary switch in the signal transduction of most growth factor receptors including EGFRs, MET or KIT. This family has three members, KRAS, HRAS and NRAS, the genes on different chromosomes. KRAS gene was identified as a homologue of the Kirsten rat sarcoma virus responsible for malignant transformation of rodent cells. [1-3] The human KRAS gene is located on chromosome 12p12.1 encoded by 6 exons. The "KRAS protein" (or GTPase KRas, EC 3.6.5.2) is evenly expressed by most tissues, but overexpressed only by a few: skeletal muscle and myocardium, uterus, adrenal cortex and certain bone marrow stem cells, otherwise rarely involved in KRAS-related carcinogenesis.[ https://www.proteinatlas.org/] RAS oncogenes are the most frequently mutated genes in human cancer. KRAS is the most frequently mutated oncogene in humans: more than 80% of pancreatic cancers, more than 30% of colorectal, cholangial cancers and lung adenocarcinomas harbor activating mutations of the KRAS gene.

*Pan-KRAS inhibitors*

Direct pan-KRAS inhibitors are reported to show activity against a broad range of KRAS-driven cell lines, including KRASG12C-, KRASG12D-, KRASG12V-, and KRASG13D-driven cells, whereas HRAS- and NRAS-mutated cell lines did not exhibit sensitivity (Kraut N. Expanding the reach of precision oncology by drugging all KRAS mutants. In: Proceedings of the 112th Annual Meeting of the American Association for Cancer Research; 2021 May 17-21. Philadelphia (PA): AACR; 2021.) BI-2852 induces inactive KRAS homodimers and selectively inhibits KRASG12D over the wild-type allele and other mutants. BI-pan-KRAS1-4 is selective for G12D and G12V, while BI-pan-KRASdegrader1 inhibited all mutant forms *in vitro.* RSC-1255 and RMC-6236 are also reported to have pan-KRAS activity.

KRASG12D is the predominant mutation type in KRAS mutant cancers and e.g. in colorectal cancer and pancreatic ductal adenocarcinoma. MRTX1133 is a selective inhibitor of KRASG12D, blocking the GTP-bounding form of the protein, while TH-Z827 blocks the GDP-bound protein (Mao, Z., Xiao, H., Shen, P. et al. KRASG12D can be targeted by potent inhibitors via formation of salt bridge. Cell Discov 8, 5 (2022). https://doi.org/10.1038). KRAS12D1-3 and KRAS12D1-3 are other reported selective inhibitors of KRASG12D (Hofman MH et al. Expanding the reach of precision oncology by drugging all KRAS mutants. Cancer Discovery 12:924,2022).

The KRASG12C mutation is the predominant KRAS mutation type in lung adenocarcinoma (LUAD), relatively frequent in cholangial and endometrial cancers and rare in colorectal or pancreatic cancers.

Allele specific irreversible inhibitors of KRAS G12C: G12C mutation and the new cysteine residue offered a relatively easy chemical target for development of covalent irreversible inhibitors which now reached the clinical testing phase. These inhibitors lock mutant KRAS in the GDP-bound state, block SOS-mediated nucleotide exchange and KRAS association with RAF [4]. The first clinically tested G12C inhibitor is AMG510/sotorasib, approved by FDA in lung cancer. This inhibitor in monotherapy exhibited good efficacy in LUAD patients but was relatively less effective in colorectal cancer patients without rate limiting toxicities [5]. Unfortunately, resistance to sotorasib treatment in the clinical setting is apparent: all patients who achieved an objective response ultimately progressed on treatment with sotorasib in a phase II study (Skoulidis F, Li BT, Dy GK, Price TJ, Falchook GS, Wolf J, et al. Sotorasib for lung cancers with KRAS p.G12C mutation. N Engl J Med 2021;384:2371-81.) The second G12C specific inhibitor entered clinical trial is MRTX849/adagrasib, which exhibits clinical activity in LUAD and colorectal cancer patients [6]. Another inhibitor, JNJ-74699, was derived from ARS853 [7] and was tested clinically but shown heavy toxicity. Its development was suspended similarly to another G12C inhibitor, LY3499446 [8]. Studies indicated that upon the treatment of cancer cells with G12C inhibitors resistance develop due to either loss of NF1 gene or one of the RAS isoforms NRAS or HRAS [7].

Another approach to inhibit a mutant KRAS protein is to lock it in a covalent complex with chaperons like cyclophyllin A. The resulting protein complex is unable to bind the SOS protein and RBD-containing RAS effector proteins. Such an inhibitor is RM007, which specifically binds to G12C KRAS.

Allele-selective KRASG12C inhibitors achieve more modest response rates than tyrosine kinase inhibitors targeting other oncogene-driven cancers due to several reasons, among them a number of heterogeneous mechanisms of resistance to these inhibitors, e.g. secondary mutations of KRAS.

Suggested combination therapies targeting the G12C mutation are G12C inhibitors combined with EGFR inhibitors, CDK4/6 MEK, mTOR, and recently, SHP2 and SOS1 inhibitors. The literature is silent on the efficacy of combinations of KRAS inhibitors and FTI compounds.

*Farnesyl transferase inhibitors (FTIs)*

Farnesylation of mutant KRAS by farnesyltransferase was long considered as a drug target. Several types of FTIs have been developed decades ago and tested clinically in various cancer types where RAS mutations are frequent, but all failed due to the alternative adaptation of RAS processing by the use of geranylgeranylation [7, 8, 9-11]. EP1165078 discloses a combination treatment for cancer, wherein an FTI compound and an additional Ras signaling pathway inhibitor induce a synergistic level of cancer cell death, however the KRAS mutation is not mentioned. WO 2021/097207 lists combinations of a KRAS G12C inhibitor with a vast number of other anticancer drugs, among them tipifarnib. No proof of any effect of either the FTI compound alone or in combination with the KRAS G12C inhibitor is provided. KRAS mutant tumors proved to be insensitive to FTIs [9-11]. On the other hand, it was demonstrated that HRAS mutant tumors are unable to use the alternative geranylgeranylation pathway [7]. Accordingly, the use of FTIs is suggested in the treatment of HRAS mutant tumors, while not considered in KRAS mutant tumors.

Despite initial promising results, tipifarnib showed no significant antitumor activity or objective response rates in non-small-cell lung cancer, small-cell lung cancer or breast cancer (A.A. Adje et al. Phase II study of the farnesyl transferase inhibitor R115777 in patients with advanced non-small-cell lung cancer. J Clin Oncol, 21 (9) (2003), pp. 1760-1766, 10.1200/JCO.2003.09.075; J.V. Heymach, et al. Phase II study of the farnesyl transferase inhibitor R115777 in patients with sensitive relapse small-cell lung cancer. Ann Oncol, 15 (8) (2004), pp. 1187-1193, 10.1093/annonc/mdh315; C. Yam, et al. A phase II study of tipifarnib and gemcitabine in metastatic breast cancer. Invest New Drugs, 36 (2) (2018), pp. 299-306, 10.1007/s10637-018-0564-2). Lonafarnib showed no objective responses in colorectal cancer and does not improve progression-free or overall survival in ovarian cancer in combination with chemotherapy (S. Sharma, et al. A phase II trial of farnesyl protein transferase inhibitor SCH 66336, given by twice-daily oral administration, in patients with metastatic colorectal cancer refractory to 5-fluorouracil and irinotecan. Ann Oncol, 13 (7) (2002), pp. 1067-1071, 10.1093/annonc/mdf173; W. Meier, et al. Randomized phase II trial of carboplatin and paclitaxel with or without lonafarnib in first-line treatment of epithelial ovarian cancer stage IIB-IV. Gynecol Oncol, 126 (2) (2012), pp. 236-240, 10.1016/j.ygyno.2012.04.050)

Recent prior art agree that FTI compounds have no clinical effect on KRAS mutation bearing cancers (Ghimessy, A. et al. Current therapy of KRAS-mutant lung cancer. Cancer Metastasis Rev 39, 1159-1177 (2020). https://doi.org/10.1007/s10555-020-09903-9), due to geranylgeranylation, and therefore they should be used for cancers with a HRAS mutation [7, 8-11, 14-18] but not for cancers with a KRAS mutation. Document

NAGASAKA MISAKO ET AL: "KRAS Inhibitors- yes but what next? Direct targeting of KRAS- vaccines, adoptive T cell therapy and beyond",CANCER TREATMENT REVIEWS, ELSEVIER, AMSTERDAM, NL, vol. 101, 21 October 2021 (2021-10-21), XP086859462,ISSN: 0305-7372 describes the present KRAS mutation carrying cancers, such as KRAS 12C variants, which are well known for being successfully treated with KRAS inhibitors. Synergistic combinations are known to be sought.

## SUMMARY OF THE INVENTION

It has been surprisingly found that farnesyl transferase inhibitor (FTI) compounds increase the anticancer effect of KRAS mutation inhibitor compounds on cancer cells with a KRAS mutation. The invention is defined by the claims. The subject matter defined in more general way is part of the general disclosure but is not part of the claimed invneiton unless it is claimed.

A farnesyl transferase inhibitor (FTI) compound according to the claims for use in the treatment of cancer is provided, wherein the FTI compound is used in combination with a KRAS inhibitor compound according to the claims.

A KRAS inhibitor compound for use in the treatment of cancer cancer is provided, wherein the KRAS inhibitor compound is used in combination with a farnesyl transferase inhibitor (FTI) compound.

Preferably the cancer cells carry a KRAS mutation.

Preferably the cancer is selected from lung cancer, colorectal cancer and pancreatic cancer.

Preferably the FTI compound and the KRAS inhibitor compound are administered concurrently or sequentially to a patient in need thereof.

A pharmaceutical composition is provided, comprising an FTI compound, a KRAS inhibitor compound and at least one pharmaceutically acceptable excipient. Preferably the pharmaceutical composition is for use in the treatment of a KRAS mutation carrying cancer.

A kit is provided, comprising a pharmaceutical composition comprising an FTI compound and at least one pharmaceutically acceptable excipient and another pharmaceutical composition comprising a KRAS inhibitor compound and at least one pharmaceutically acceptable excipient. Preferably the kit is for use in the treatment of a KRAS mutation carrying cancer.

The FTI compound and the KRAS inhibitor compound are administered to a patient having a KRAS mutant cancer, in amounts effective to induce a synergistic effect on the cancer cells.

Preferably the KRAS inhibitor compound is administered in a dose effective in the treatment of cancer, preferably a KRAS mutant cancer.

Preferably the KRAS inhibitor compound is selected from a KRASG12D inhibitor, a KRASG12C inhibitor and a pan-KRAS inhibitor.

More preferably the KRAS inhibitor compound is a KRASG12D inhibitor.

More preferably the KRAS inhibitor compound is a KRASG12C inhibitor, preferably an allele specific irreversible inhibitor of KRAS-G12C.

More preferably the KRAS inhibitor compound is a pan-KRAS inhibitor.

Preferably the mutation is selected from KRAS G12D, G12C, G12V, G12S and G13D. More preferably the mutation is KRAS G12D. More preferably the mutation is KRAS G12C. More preferably the mutation is KRAS G12V. More preferably the mutation is KRAS G12S. More preferably the mutation is KRAS G13D.

The KRAS inhibitor compound is selected from MRTX1133, BI2852, sotorasib, adagrasib and ARS1620. The KRAS inhibitor compound is preferably selected from sotorasib and adagrasib. More preferably the KRAS inhibitor compound is MRTX1133. More preferably the KRAS inhibitor compound is BI2852. More preferably the KRAS inhibitor compound is sotorasib. More preferably the KRAS inhibitor compound is adagrasib. More preferably the KRAS inhibitor compound is ARS1620.

The FTI compound is selected from tipifarnib, lonafarnib, FTI277 and BMS214662. The FTI compound is preferably selected from tipifarnib and lonafarnib. The FTI compound is more preferably tipifarnib, more preferably lonafarnib, more preferably FTI277 or more preferably BMS214662.

Preferably the cancer is selected from lung cancer, colorectal cancer and pancreatic cancer. More preferably the cancer is lung cancer. More preferably the cancer is colorectal cancer. More preferably the cancer is pancreatic cancer.

Preferably the mutation is KRAS G12D, and the FTI compound is selected from tipifarnib and lonafarnib. More preferably the mutation is KRAS G12D, the KRAS inhibitor compound is MRTX1133 and the FTI compound is selected from tipifarnib and lonafarnib. Preferably the mutation is KRAS G12D and the KRAS inhibitor compound is MRTX1133.

Preferably the mutation is KRAS G12C and the FTI compound is selected from tipifarnib, lonafarnib, FTI277 and BMS214662, preferably from tipifarnib and lonafarnib. More preferably the mutation is KRAS G12C and the FTI compound is selected from tipifarnib, lonafarnib, FTI277 and BMS214662, preferably from tipifarnib and lonafarnib and the KRAS inhibitor compound is selected from sotorasib and adagrasib, ARS1620 and BI2852, preferably from sotorasib and adagrasib. More preferably the mutation is KRAS G12C, the FTI compound is tipifarnib and the KRAS inhibitor compound is sotorasib. More preferably the mutation is KRAS G12C, the FTI compound is tipifarnib and the KRAS inhibitor compound is adagrasib. More preferably the mutation is KRAS G12C, the FTI compound is lonafarnib and the KRAS inhibitor compound is sotorasib. More preferably the mutation is KRAS G12C, the FTI compound is lonafarnib and the KRAS inhibitor compound is adagrasib. Preferably the mutation is KRAS G12C and the KRAS inhibitor compound is selected from sotorasib and adagrasib, ARS1620 and BI2852, preferably from sotorasib and adagrasib.

Preferably the mutation is selected from KRAS G12V, KRAS G12S and KRAS G13D, preferably the mutation is KRAS G12V, preferably the mutation is KRAS G12S, preferably the mutation is KRAS G13D and the KRAS inhibitor compound is a pan-KRAS inhibitor. Preferably the mutation is selected from KRAS G12V, KRAS G12S and KRAS G13D, the KRAS inhibitor compound is a pan-KRAS inhibitor and the FTI compound is selected from tipifarnib or lonafarnib. Preferably the mutation is selected from KRAS G12V, KRAS G12S and KRAS G13D, preferably the mutation is KRAS G12V, preferably the mutation is KRAS G12S, preferably the mutation is KRAS G13D and the FTI compound is selected from tipifarnib or lonafarnib.

Preferably the daily dose of the KRAS inhibitor compound is 1-25 mg/kg body weight, more preferably 4-10 mg/kg body weight. Preferably the daily dose of the FTI compound is 10-80 mg/kg body weight, more preferably 35-45 mg/kg body weight.

In some embodiments, the FTI is administered at a dose of 50-2400 mg daily. In some embodiments, the FTI is administered at a dose of 100-1800 mg daily. In some embodiments, the FTI is administered at a dose of 100-1400 mg b.i.d. In some embodiments, the FTI is administered at a dose of 100-1200 mg b.i.d. For example, lonafarnib may be administered orally in a dose of 150mg/m$^2$ twice a day, tipifarnib may be administered orally in a dose of about 600 mg 2x daily, sotorasib may be administered orally in a dose of about 960 mg/day and adagrasib may be administered orally in a dose of about 600 mg 2x daily. The skilled person will understand that the dose to be administered, the route to be chosen for administration and the duration of the treatment depend on the individual and the cancer to be treated, as well as on the specific FTI compound and KRAS inhibitor compound used. Preferably the usual clinical therapeutic doses of the drugs are to be used, or a dose lower than the therapeutic dose of the specific compound when given alone or in combination with other anti-cancer treatments.

For administration to a human in the treatment of the cancer typical dosages of the KRAS inhibitor can be about 0.05 mg/kg/day to about 50 mg/kg/day, or 2.5 mg/day to about 5000 mg/day. For example, can be about 0.005 mg/kg/day to about 50 mg/kg/day, about 0.1 mg/kg/day to 10 mg/kg/day, about 1 mg/kg/day to about 5 mg/kg/day, about 1 mg/day to about 1000 mg/day, about 20 mg/day to about 750 mg/day, about 3 mg/day to about 500 mg/day, about 5 mg/day to about 250 mg/day, about 10 mg/day to about 100 mg/day, about 3 mg/day to about 10 mg/day, or about 100 mg/day to about 250 mg/day. The doses may be administered as single dose or may be divided into multiple doses.

## BRIEF DESCRIPTION OF THE FIGURES

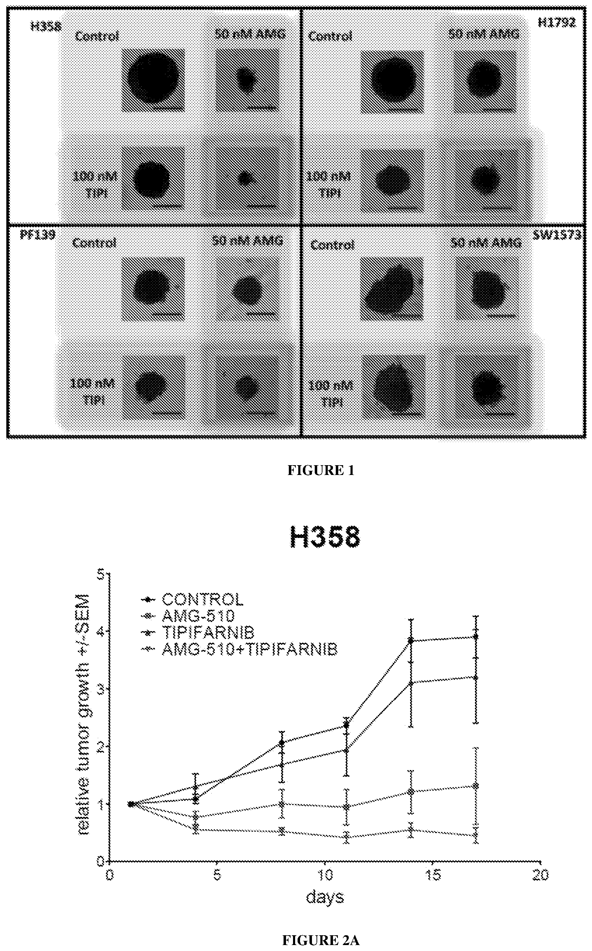

*Figure 1*. Three-dimensional spheroid growth experiment. Representative pictures show the effect of the inhibitors on the sixth day after treatment. Scale bar means 200 μm.

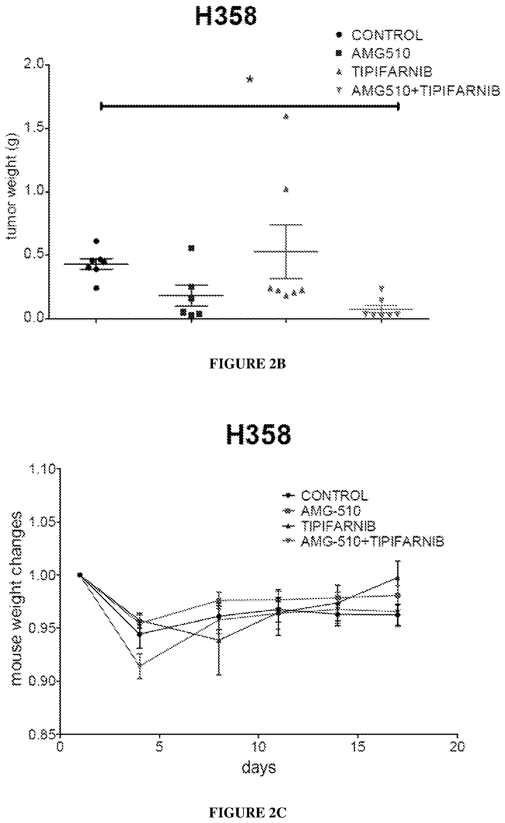

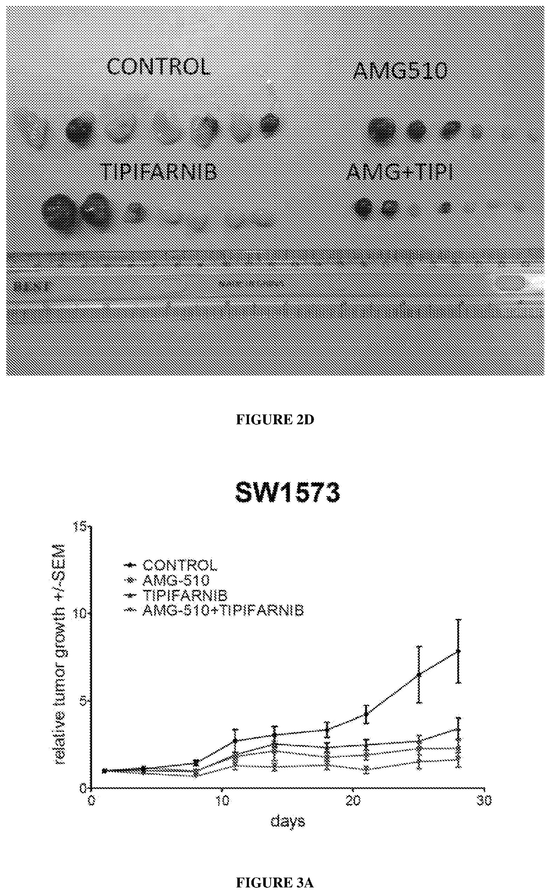

*Figure 2. In vivo* tumor growth of H358 xenografts upon sotorasib/AMG510 (5 mg/kg i.p.) and/or tipifarnib (40 mg/kg i.p.) therapy. **(A)** Tumor volume was determined twice/weekly using caliper. Relative tumor volume growth are shown on the graph. **(B)** Tumor weights (gram) of H358 xenografts at day18. (C) Mouse weight changes. (D) Picture of the harvested tumors. Only combinational treatment resulted in significant effect compared to control.

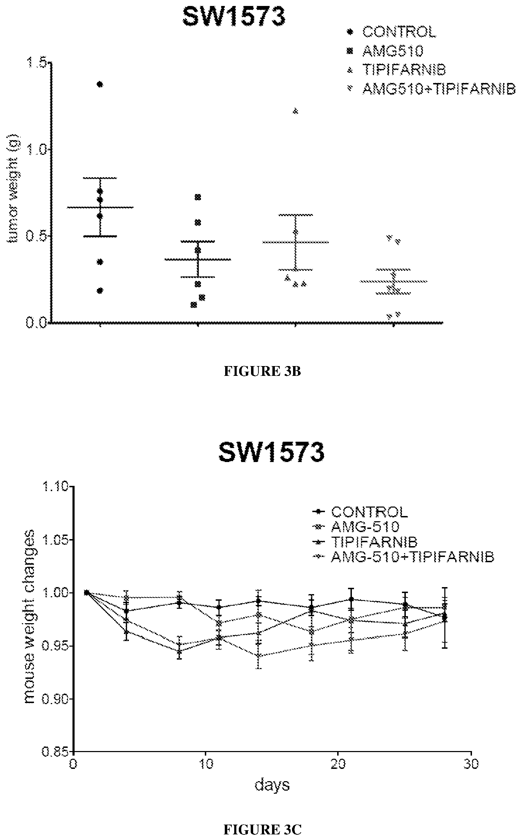

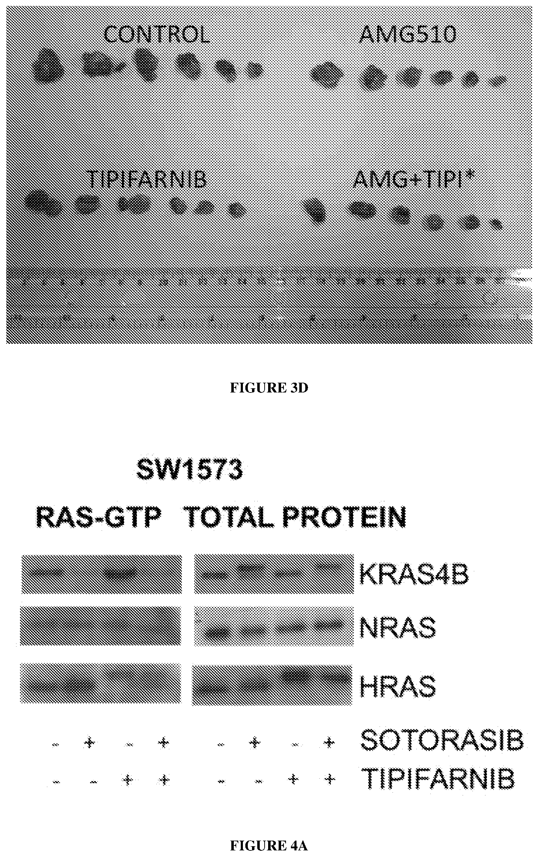

*Figure 3. In vivo* tumor growth of SW1573 xenografts upon sotorasib/AMG510 (25 mg/kg i.p.) and/or tipifarnib (40 mg/kg i.p.) therapy. **(A)** Tumor volume was determined twice/weekly using caliper. Relative tumor volume growth are shown on the graph. **B** Tumor weights (gram) of SW1573 xenografts at day28. (C) Mouse weight changes. **(D)** Picture of the harvested tumors (*the smallest tumor of the AMG510+tipifarnib group was not included by accident).

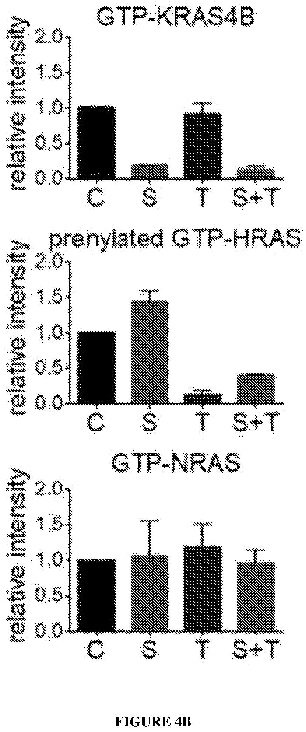

*Figure 4.* Changes in RAS protein expression and activation following 48-hour-long treatment with sotorasib (100 nM) and/or tipifarnib (500 nM) a) Images show representative blots of the specified RAS proteins. RAS-GTP stands for only GTP-bound, active RAS proteins while total protein shows blots from the whole cell lysates. b) Graphs represents normalized level of GTP-bound KRAS4B, HRAS and NRAS, respectively. Note that graph shows only changes of prenylated fraction of HRAS. Protein levels were normalized to control. All data shown derives from three independent experiments. Graphs shows mean data with error bars representing SEM.

## DETAILED DESCRIPTION OF THE INVENTION

It has been surprisingly found that FTIs exert a beneficial antitumoral effect on KRAS mutant cancer cells when given in combination with a KRAS mutant inhibitor. It has been thus demonstrated that FTIs are effective in KRAS mutant cancers when given in combination with a KRAS mutant inhibitor, despite the long standing understanding that FTIs have no or very limited effects in the treatment of cancers with a KRAS mutation due to RAS processing using geranylgeranylation if the farnesyl transferase is blocked. Accordingly, a novel combination therapy to treat KRAS mutant cancers is herein provided.

Combined treatment of KRAS mutant cancer cell lines with a KRAS inhibitor and an FTI proved that KRAS inhibitors and FTIs potentiate each others effect on KRAS mutation bearing cancer cells.

*Effect of the combination of a KRASG12C inhibitor and an FTI on KRASG12C mutant cancer*

Synergistic antitumor effect of sotorasib and tipifarnib, sotorasib and lonafarnib, sotorasib and FTI277, sotorasib and BMS214662 was demonstrated on KRASG12C mutant human lung cancer, colon cancer and pancreatic cancer lines.

An FTI compound for use in the treatment of a KRAS12C mutant cancer is provided, wherein the FTI compound is used in combination with a KRASG12C inhibitor compound, wherein the FTI compound is preferably selected from tipifarnib, lonafarnib, FTI277 and BMS214662, more preferably from tipifarnib and lonafarnib, more preferably the FTI compound is tipifarnib or more preferably the FTI compound is lonafarnib, the KRASG12C inhibitor compound is preferably selected from sotorasib and the cancer is preferably selected from lung cancer, colorectal cancer and pancreatic cancer.

Synergistic antitumor effect of adagrasib and tipifarnib, adagrasib and lonafarnib was demonstrated on KRASG12C mutant human lung cancer, colon cancer and pancreatic cancer lines.

An FTI compound for use in the treatment of a KRAS12C mutant cancer is provided, wherein the FTI compound is used in combination with a KRASG12 inhibitor compound, wherein the FTI compound is preferably selected from tipifarnib and lonafarnib, the KRAS12C inhibitor compound is preferably adagrasib and the cancer is preferably selected from lung cancer, colorectal cancer and pancreatic cancer.

Synergistic antitumor effect of ARS1620 and tipifarnib was demonstrated on KRASG12C mutant human lung cancer.

An FTI compound for use in the treatment of a KRAS12C mutant cancer is provided, wherein the FTI compound is used in combination with a KRASG12 inhibitor compound, wherein the FTI compound is preferably tipifarnib, the KRAS12C inhibitor compound is preferably ARS1620 and the cancer is preferably lung cancer.

*Effect of the combination of a KRASG12D inhibitor and an FTI on KRASG12D mutant cancer*

Synergistic antitumor effect of MRTX1133 and tipifarnib, MRTX1133 and lonifarnib was demonstrated on KRASG12D mutant human lung cancer, colon cancer and pancreatic cancer lines.

An FTI compound for use in the treatment of a KRAS12D mutant cancer is provided, wherein the FTI compound is used in combination with a KRASG12 inhibitor compound, wherein the FTI compound is tipifarnib or lonafarnib, the KRAS12C inhibitor compound is MRTX1133 and the cancer is preferably lung cancer, colorectal cancer or pancreatic cancer.

*Effect of the combination of a pan-KRAS inhibitor and an FTI on tumors carrying different KRAS mutations*

Synergistic antitumor effect of BI2852 and tipifarnib was demonstrated on KRAS G12D, G12V, G12S and G13D mutant human lung cancer, colon cancer and pancreatic cancer lines, while the synergistic antitumor effect of BI2852 and lonafarnib was demonstrated on KRAS G12V mutant human colon cancer.

An FTI compound for use in the treatment of a KRASG12C mutant cancer is provided, wherein the FTI compound is used in combination with a pan-KRAS inhibitor compound, wherein the FTI compound is tipifarnib, the pan-KRAS inhibitor compound is BI2852 and the cancer is preferably lung cancer.

An FTI compound for use in the treatment of a KRASG12D mutant cancer is provided, wherein the FTI compound is used in combination with a pan-KRAS inhibitor compound, wherein the FTI compound is tipifarnib, the pan-KRAS inhibitor compound is BI2852 and the cancer is preferably pancreatic cancer or preferably colorectal cancer.

An FTI compound for use in the treatment of a KRASG12V mutant cancer is provided, wherein the FTI compound is used in combination with a pan-KRAS inhibitor compound, wherein the FTI compound is tipifarnib or lonafarnib, the pan-KRAS inhibitor compound is BI2852 and the cancer is preferably colorectal cancer.

An FTI compound for use in the treatment of a KRASG12S mutant cancer is provided, wherein the FTI compound is used in combination with a pan-KRAS inhibitor compound, wherein the FTI compound is tipifarnib, the pan-KRAS inhibitor compound is BI2852 and the cancer is preferably lung cancer.

An FTI compound for use in the treatment of a KRAS13D mutant cancer is provided, wherein the FTI compound is used in combination with a pan-KRAS inhibitor compound, wherein the FTI compound is tipifarnib, the pan-KRAS inhibitor compound is BI2852 and the cancer is preferably colorectal cancer.

Preferably the FTI compound and the KRAS inhibitor compound are each administered in a dose effective in the treatment of cancer.

Preferably the FTI compound is administered in a dose effective in the treatment of cancer.

The term "synergistic" and the term "potentiating" as used herein refer to the term introduced to the scientific community by Cho 2006 (DOI: 10.1124/pr.58.3.10), using Combination Index values as indicators for drug interactions. More precisely, CI values less then 1 indicates synergistic interactions. The Combination Index is used to determine the degree of drug interaction. Its formula is the sum of the ratio of the dose of each drug in the compound to the dose when used alone when the combination and compound produce 50% efficacy.

The formula is

$$CI = \frac{D1}{(Dx)1} + \frac{D2}{(Dx)2}$$

wherein
(Dx)1 and (Dx)2 represent the concentration of each drug alone to exert x% effect alone, while D1 and D2 are the concentrations of drugs in combination to elicit the same effect. CI < 1, = 1 or > 1 indicates synergism, additivity or antagonism, respectively.

As used herein, "treatment" (including variations thereof, e.g., "treat" or "treated") means one or more of the following: (i) the reduction in the severity of a disease, (ii) alleviation or elimination of symptoms of a disease, and (iii) the substantial or complete elimination of the disease. Treatment may refer to therapeutic treatment (i.e. treatment when the disease, e.g. cancer cells is already manifested in the patient). Treatment may also refer to prophylactic treatment when the aim of the treatment is at least in part to prevent or mitigate the formation of metastases or the recurrence of a tumor. The compounds for use, composition, kits and methods described herein are useful for inhibiting the growth of tumors expressing an activated ras oncogene.

Cancer malign neoplasm and is a type of abnormal and excessive growth of tissue. The growth of cancer is uncoordinated with that of the normal surrounding tissue, and persists in growing abnormally, even if the original trigger is

EP 4 426 303 B1

removed. This abnormal growth usually forms a mass, when it may be called a tumor.

A number of cancers are listed in Kumar, V., Abbas, A., and Aster, J. (2017). Basic pathology (Elsevier). A "carcinoma" is a neoplasm which arises from transformed cells of epithelial origin, including epithelial tissue of the skin, or the tissue that lines internal organs, such as the liver or kidneys. Carcinomas may be confined to the primary location of origin, however, they may also spread to other parts of the body.

The FTI compound and the KRAS (e.g. KRAS-G12C or G12D) inhibitor compound are administered simultaneously (e.g. in separate or unitary compositions) or sequentially in either order. The preferred method and order of administration and the respective dosage amounts and regimes for the FTI compound and the KRAS inhibitor compound will depend on the particular FTI and on the particular KRAS inhibitor compound being administered, the route of administration of the combination, the tumor being treated and the patient being treated. The method and order of administration and the dosage amounts can be determined by the skilled person using conventional methods. The FTI compound and the KRAS inhibitor compound may be administered continuously or intermittently.

Preferably the FTI compound and the KRAS inhibitor compound are in amounts effective to induce a synergistic effect on the cancer cells. The effect may be e.g. an inhibition in the growth of cancer cells, killing of cancer cells, and a reduction in symptoms associated with the presence of the cancer cells. A prophylactic effect may be e.g. delaying or eliminating the appearance of a proliferative disease, delaying or eliminating the onset of symptoms of the proliferative disease, slowing, halting, or reversing the progression of the proliferative disease, or any combination thereof.

"Farnesyltransferases" (EC 2.5.1.58) are enzymes which belong to the prenyltransferase group of enzymes and catalyze the addition of a farnesyl group to a cysteine residue of a C-terminal CAAX motif in various proteins involved in cell signaling including the H-Ras protein and regulate the activation of Ras protein.

The term "farnesyl transferase inhibitor" or "FTI" refers to any compound which potently inhibits farnesyl transferase (but preferably not geranylgeranyl transferase).

The term "KRAS mutant inhibitor", "KRAS inhibitor", "KRAS mutant inhibitor compound" or "KRAS inhibitor compound" refers to a compound that is capable of inhibiting a mutant KRAS enzyme, thereby inhibiting signaling pathways downstream from KRAS.

The term "allele specific inhibitor of KRASG12C" and "direct KRASG12C inhibitor" refers to compounds which act by selectively forming a covalent bond with cysteine 12 within the switch-II pocket of KRAS-G12C protein, thereby locking KRAS in the inactive state. Similarly, the term "allele specific inhibitor of KRAS [mutation]" and "direct KRAS [mutation] inhibitor" refers to compounds which act by selectively forming a bond with the [mutated amino acid], thereby locking KRAS in the inactive state. The [mutation] may be e.g. G12C, G12D, G12V, G12S or G13D. The [mutated amino acid] may be e.g. aspartic acid 12, valine 12, serin 12 or aspartic acid 13.

In some embodiments, the FTI is tipifarnib; arglabin; perrilyl alcohol; lonafarnib; FTI-277; L744832; R208176; BMS 214662; or salirasib. Tipifarnib and lonafarnib are highly preferred. Only the FTI inhibitors according to the claims are part of the invention.

In some preferred embodiments the KRAS G12C inhibitor is sotorasib. In other preferred embodiments the KRAS G12C inhibitor is ARS1620. In yet other preferred embodiments the KRAS G12C inhibitor is adagrasib.

In some embodiments, the FTI is administered at a dose of 50-2400 mg daily. In some embodiments, the FTI is administered at a dose of 100-1800 mg daily. In some embodiments, the FTI is administered at a dose of 100-1400 mg b.i.d. In some embodiments, the FTI is administered at a dose of 100-1200 mg b.i.d. For example, lonafarnib may be administered orally in a dose of 150mg/m$^2$ twice a day, tipifarnib may be administered orally in a dose of about 600 mg 2x daily, sotorasib may be administered orally in a dose of about 960 mg/day and adagrasib may be administered orally in a dose of about 600 mg 2x daily. The skilled person will understand that the dose to be administered, the route to be chosen for administration and the duration of the treatment depend on the individual and the cancer to be treated, as well as on the specific FTI compound and KRAS inhibitor compound used. Preferably the usual clinical therapeutic doses of the drugs are to be used, or a dose lower than the therapeutic dose of the specific compound when given alone or in combination with other anti-cancer treatments.

For administration to a human in the treatment of the cancer typical dosages of the KRAS (e.g. KRASG12C) inhibitor can be about 0.05 mg/kg/day to about 50 mg/kg/day, or 2.5 mg/day to about 5000 mg/day. For example, can be about 0.005 mg/kg/day to about 50 mg/kg/day, about 0.1 mg/kg/day to 10 mg/kg/day, about 1 mg/kg/day to about 5 mg/kg/day, about 1 mg/day to about 1000 mg/day, about 20 mg/day to about 750 mg/day, about 3 mg/day to about 500 mg/day, about 5 mg/day to about 250 mg/day, about 10 mg/day to about 100 mg/day, about 3 mg/day to about 10 mg/day, or about 100 mg/day to about 250 mg/day. The doses may be administered as single dose or may be divided into multiple doses.

The pharmacological composition or dosage unit comprising the FTI compound and/or the KRAS (e.g. KRASG12C) inhibitor compound may be administered orally, preferably in the form of a tablet, capsule or pill or in a liquid formulation, e.g. in the form of a continuous infusion. Pharmaceutically acceptable ingredients are well known and include binders, lubricants, surfactants, sweetening and flavoring agents, coating materials, preservatives, dyes, thickeners, adjuvants, antimicrobial agents, antioxidants and carriers.

The pharmacological composition or dosage unit comprising the FTI compound and/or the KRAS (e.g. KRASG12C) inhibitor compound may be administered by injection. Injectable compositions can be in the form of suspensions, solutions, or emulsions in oily or aqueous vehicles, and can contain formulatory agents such as suspending, stabilizing, and/or dispersing agents.

Combinational effects of farnesyl-transferase inhibitors lonafarnib and tipifarnib and KRAS G12C inhibitors AMG510 (sotorasib), ARS1620, MRTX849 (adagrasib) were tested on four KRAS G12C mutant lung adenocarcinoma cell lines *in vitro*, while *in vivo* tests were performed on mice bearing tumors induced by the injection of two human lung adenocarcinoma cell lines. Also, AMG510 and MRTX849 in combination with tipifarnib were also tested *in vitro* on pancreatic and colon cancer lines. Unexpectedly, combination of a FTI compound and a KRAS-G12C inhibitor compound resulted in a synergistic effect in all cell lines and all experimental settings, while the combinations of sotorasib with other prenylation inhibitors (statins, bisphosphonates) were less effective.

The term "treatment may include curative treatment, the ameliariation, decreasing, mitigating of, effects, duration, prevalence, severity of a disease or a symptom of a disease, as well as prophylactic treatment.

The term "comprises" or "comprising" or "including" are to be construed here as having a non- exhaustive meaning and allow the addition or involvement of further features or method steps or compo-nents to anything which comprises the listed features, for example components or ingredients in a pharmaceutical composition or kit or method steps. "Comprising" can be substituted by "including" if the practice of a given language variant so requires or can be limited to "consisting essentially of" if other members or components are not essential to reduce the invention to practice.

The singular forms "a", "an" and "the", or at least "a", "an", include plural reference unless the context clearly dictates otherwise.

Other aspects of the invention

The invention relates to a farnesyl transferase inhibitor (FTI) compound for use in the treatment of cancer, wherein the cancer cells carry a KRAS-G12C mutation and wherein the FTI compound is used in combination with a KRAS-G12C inhibitor compound, as defined in the claims.

The invention relates to a KRAS-G12C inhibitor compound for use in the treatment of cancer, wherein the cancer cells carry a KRAS-G12C mutation and wherein the KRAS-G12C inhibitor compound is used in combination with a farnesyl transferase inhibitor (FTI) compound.

In another aspect the use of a farnesyl transferase inhibitor (FTI) compound in combination with a KRAS-G12C inhibitor compound for the manufacture of a pharmaceutical composition for the treatment of cancer is provided, wherein the cancer cells carry a KRAS-G12C mutation.

Preferably the cancer is a carcinoma. Preferably the cancer is adenocarcinoma.

Preferably the cancer is lung adenocarcinoma or colon adenocarcinoma or preferably the cancer is pancreatic adenocarcinoma.

Preferably the FTI compound and the KRAS-G12C inhibitor compound are administered concurrently or sequentially to a patient in need thereof .

The invention relates to a pharmaceutical composition comprising an FTI compound, a KRAS-G12C inhibitor compound and at least one pharmaceutically acceptable excipient, for use according to the invention.

In an embodiment the composition is a veterinary composition. In a particular embodiment the pharmaceutical composition is a human medicine.

The invention relates to a pharmaceutical kit comprising a pharmaceutical dosage unit comprising an FTI compound and at least one pharmaceutically acceptable excipient and another pharmaceutical dosage unit comprising a KRAS inhibitor compound and at least one pharmaceutically acceptable excipient, for use according to the claims.

Preferably the cancer is a carcinoma. Preferably the cancer is adenocarcinoma.

Preferably the cancer is lung adenocarcinoma or colon adenocarcinoma or preferably the cancer is pancreatic adenocarcinoma.

Preferably the FTI compound and the KRAS-G12C inhibitor compound are administered to a patient having a KRAS-G12, preferably a KRAS-G12C mutant cancer, in amounts effective to induce a synergistic effect on the cancer cells. Preferably the effect is the level of cancer cell death. Preferably the FTI compound and the KRAS-G12C inhibitor compound are administered to a patient having a KRAS-G12, preferably a KRAS-G12C mutant cancer, in amounts effective to induce a synergistic (potentiating) effect on the cancer. Preferably the effect is a decrease in the number or volume of tumors and/or metastases in the patient.

Preferably the FTI compound is selected from tipifarnib and lonafarnib.

Preferably the KRAS-G12C inhibitor compound is an allele specific irreversible inhibitor of KRAS-G12C.

Preferably the KRAS-G12C inhibitor compound is selected from sotorasib, adagrasib and ARS1620.

Preferably the FTI compound is tipifarnib and the KRAS-G12C inhibitor compound is selected from sotorasib, adagrasib and ARS1620. Preferably the FTI compound is tipifarnib and the KRAS-G12C inhibitor compound is selected from sotorasib and adagrasib.

Preferably the FTI compound is lonafarnib and the KRAS-G12C inhibitor compound is selected from sotorasib, adagrasib and ARS1620. Preferably the FTI compound is lonafarnib and the KRAS-G12C inhibitor compound is selected from sotorasib and adagrasib.

Preferably, the FTI compound is administered in a dose of 50-2500 mg/day.

Preferably the KRAS-G12C inhibitor compound is administered in a dose of 100-2000 mg/day.

Preferably the patient is a vertebrate animal, in particular a mammal, more particularly a human patient.

EXAMPLES

Materials and methods

*Cells and reagents*

Combinational effects of farnesyl-transferase inhibitors and different KRAS-targeting inhibitors (showed in Table1) were tested on human cancer cell lines with different KRAS mutant status of lung, colorectal and pancreatic tissue of origin shown in Table2.. H358, SW1573, H1792, MIAPACA2, SW1990, , SNUC2B, SW480, A549 and HCT116 were purchased from ATCC, while SW48 KRAS G12C colorectal cell line and H838 KRAS G12D lung cell line was bought from Horizon Discovery Ltd. Most of the cell lines were heterozygous for mutant KRAS, as in human cases used to occur, but we also used cell lines with homozygous mutant KRAS.

Table 1. Farnesyl-transferase inhibitors and different KRAS-targeting KRAS G12C inhibitors used

| AGENT | TARGET |
|---|---|
| SOTORASIB | KRAS G12C |
| ADAGRASIB | KRAS G12C |
| ARS1620 | KRAS G12C |
| MRTX1133 | KRAS G12D |
| BI2852 | PAN-KRAS |
| TIPIFARNIB | FARNESYL-TRANSFERASE ENZYME |
| LONAFARNIB | FARNESYL-TRANSFERASE ENZYME |
| FTI277 | FARNESYL-TRANSFERASE ENZYME |
| BMS214662 | FARNESYL-TRANSFERASE ENZYME |

Table 2. Cell lines used in the experiments

| HUMAN CANCER CELL LINE | TISSUE OF ORIGIN | KRAS MUTANT STATUS |
|---|---|---|
| H358 | LUNG | KRAS G12C |
| H1792 | LUNG | KRAS G12C |
| PF139 | LUNG | KRAS G12C |
| SW1573 | LUNG | KRAS G12C |
| PF97 | COLORECTAL | KRAS G12C |
| SW48 KRAS G12C | COLORECTAL | KRAS G12C (genetically modified cell line) |
| MIAPACA2 | PANCREAS | KRAS G12C |
| SW1990 | PANCREAS | KRAS G12D |
| H838 KRAS G12D | LUNG | KRAS G12D (genetically modified cell line) |
| PF906 | LUNG | KRAS G12D |
| SNUC2B | COLORECTAL | KRAS G12D |
| SW480 | COLORECTAL | KRAS G12V |

(continued)

| HUMAN CANCER CELL LINE | TISSUE OF ORIGIN | KRAS MUTANT STATUS |
|---|---|---|
| A549 | LUNG | KRAS G12S |
| HCT116 | COLORECTAL | KRAS G13D |

PF139 lung adenocarcinoma, PF97 colorectal adenocarcinoma and PF906 lung adenocarcinoma cell lines were developed by B Hegedűs in Essen from pleural effusion. Cells were cultured in DMEM (Lonza, Switzerland; with 4500 mg/dm3 glucose, pyruvate and L-glutamine) supplemented with 10% fetal calf serum (EuroClone) and 1% penicillin-streptomycin-amphotericin (Lonza) in tissue culture flasks in a humidified 5% $CO_2$ atmosphere at 37 °C.

Lonafarnib, tipifarnib for *in vitro* experiments were purchased from Sigma, while AMG510, ARS1620, MRTX849, MRTX1133, BI2852 and tipifarnib for the animal experiments were obtained from Medchemexpress. For *in vitro* experiments drugs were dissolved in DMSO in 10 mM stock concentration and stored at -80 °C. Farnesyl-transferase inhibitors lonafarnib and tipifarnib and a statin drug as an inhibitor of the mevalonate pathway, simvastatin for *in vitro* experiments were purchased from Sigma, while KRAS-G12C allele specific irreversible inhibitors AMG510 (sotorasib), ARS1620, MRTX849 (adagrasib), MRTX1133, BI2852 and tipifarnib for the animal experiments were obtained from Medchemexpress. BPH1222 (a farnesyl-pirophosphate-synthase inhibitor, a variant of N-bisphosphonate as prenylation inhibitor) was synthetized in at the Department of Organic Chemistry, Eötvös Loránd University as described in [12]. For *in vitro* experiments drugs were dissolved in DMSO in 10 mM stock concentration and stored at -80 °C.

*2D combinational tests*

Cells that reached 70-80% confluency were trypsinized and counted using Luna II Automated Cell Counter. Cells were plated in 5000-10000 cells/well density (depending on the growth rate of the given cell line) on a 24 well plate. Next day the medium was replaced to fresh medium supplemented with the inhibitors. After 6 days, the wells were washed with DPBS (Lonza) and the cells were fixed by 10% trichloroacetic acid and stained with Sulphorodamine-B (SRB) (Sigma) dye for 15 minutes. Plates then were repeatedly washed with 1% acetic acid to remove excess dye. Protein-bound SRB was then dissolved in 10 mM Tris buffer (pH=7,4) and OD was measured at 570 nm using a microplate reader (EL800, BioTec Instruments, Winooski, VT). OD values were normalized to control. Transformed data then were used to calculate combinational index (CI) values using Compusyn software as described in https://www.combosyn.com/index.html. Data shown are results of three independent experiments.

*3D spheroid tests*

Spheroids were generated using polyHEMA coated 96well round bottom plates. Briefly, the inner 60 wells of the plate were coated with 60 μl 5 mg/ml polyHEMA dissolved in 96% ethanol. Plates were dried at 60 °C in a plate shaker while shaking, then sterilized with UV. Cells were seeded at 3000 cells/well density at the inner 60 polyHEMA coated wells in 200 μl medium, while the outer wells were filled with DPBS (Lonza) to avoid evaporation. Plates were then centrifugated at 2000 RPM so that cells would be concentrated at the bottom of the well thus enhancing single spheroid formation. Within 24 hours the cells aggregated into spheroids and were treated by adding further 100 μl medium supplemented with the inhibitors. Each concentration group contained three spheroids. Each spheroid was pictured using a ToupCam XW 3MP microscope-camera on the first and sixth day of the treatment. Before imaging, medium was gently suspended so that unattached or dead cells would be removed from the surface of the spheroids. Images then were analysed using ImageJ software with a modified script of [1]. Briefly, the script measures the area of the 2D projections of the spheroids. Results were manually reviewed, then area values were used to calculate radius and volume of spheroids using the formula $V=4/3 \times \pi \times$ radius$^3$. Data are shown as results of three independent experiments.

*Western blotting*

RAS protein levels (whole lysate) was investigated by western blot analyses. Following 48 hours treatment with 500 nM Tipifarnib, 100 nM AMG510 and their combination in 6-well plates, cells were washed with DPBS and fixed with 6% trichloroacetic acid for an hour at 4°C. Cells then were mechanically harvested, centrifuged. Precipitated protein was dissolved in modified Läemmli-type sample buffer containing 0.02% bromophenol blue, 10% glycerol, 2% SDS, 100 mM dithiothreitol (DTT), 5 mM EDTA, 125 mg/ml urea, 90 mM Tris-HCl, pH 7.9. Qubit Fluorometer was used for determination of Protein concentration. Equal amounts of protein were loaded onto 10% poliacrylamid gels and transferred after electrophoretic separation to PVDF membranes. Analyses of RAS levels were performed using KRAS4B antibody (WH0003845M1, Sigma), HRAS antibody (18295-1-ap, Proteintech) and NRAS antibody (SC-31, Santa Cruz Biotechnology). All antibodies were dissolved according to the manufacturer instructions in 5% BSA or dry milk in 1x TTBS buffer. Membranes were blocked at room temperature in 5% dry milk dissolved in 1x TTBS for an hour, then were incubated in primary antibodies overnight at 4°C. HSP conjugated rabbit secondary antibodies (1:10000, 1 h, RT) and Pierce ECL Western Blotting Substrate (Thermo Scientific) were used for visualization. Ponceau staining was used for normalization. Quantification was performed using ImageJ software. Each cell line was analyzed in 3 biological replicates.

*Active RAS pulldown experiments*

GTP-bound RAS samples were prepared using Active Ras Pull-Down and Detection Kit (Cat.no: 16117) from Thermo Fisher Scientific. SW1573 cells were placed in 6-well plates and left for a day to attach to surface. Next day medium was replaced to fresh medium containing the inhibitors. Following 48 hours treatment with 500 nM tipifarnib, 100 nM sotorasib or their combination in 6-well plates, cells were isolated according to the manufacturer's instruction. Briefly, cells were lysed with Lysis/Binding/Wash buffer containing proteinase inhibitor and lysates were centrifuged at 16000xg. Supernatant was transferred to a new Eppendorf tube and protein concentration was determined with Qubit Fluorometer. 1200 $\mu$g from each sample were then incubated with GST-Raf1-RBD and Glutathione Agarose Resin for an hour at room temperature and was centrifuged in a spin cup. Trapped GTP-bound RAS protein was removed from the spin column and dissolved in 50 $\mu$l DTT containing 2X SDS sample buffer. Equal volumes of each samples were then loaded onto 10% poliacrylamid gels and transferred after electrophoretic separation to PVDF membranes. Signal development was performed as stated in the above section. Quantification was performed using ImageJ software. Each cell line was analyzed in 3 biological replicates.

*In vivo experiments*

H358 and SW1573 human lung adenocarcinoma cells ($5\times10^6$ and $1\times10^6$, respectively) were subcutaneously injected in female SCID mice. Cells were injected in 200 $\mu$l DMEM:Matrigel mixture (ratio 1:1) based on preliminary experiments. When tumor reached approximately 100 mm³ (H358 7 days, SW1573 26 days after injection), animals were randomized and treated intraperitoneally daily except for weekends. Drugs were dissolved in 60% DPBS, 34% PEG300, 5% DMSO and 1% Tween80. H358 xenografts were treated with 5 mg/kg AMG510; 40 mg/kg Tipifarnib, while SW1573 xenografts were treated with 25 mg/kg AMG510 and 40 mg/kg Tipifarnib. Controls received vehicle. Doses were determined based on previous literature, roughly using half of the used *per os* doses. The subcutaneous tumors were measured with a caliper and tumor volumes were calculated with the formula $V=4/3\pi \times (length \times width^2)$ and expressed in mm³. H358 experiment was terminated after 18 days and SW1573 after 25 days treatment. Tumors were measured upon harvest, then fixed with 4% PFA for histological analyses.

Animals were the properties of the Department of Experimental Pharmacology, National Institute of Oncology, H-1122, Budapest, Hungary. All experiments were carried out in accordance with the Guidelines for Animal Experiments and were approved for the Department of Experimental Pharmacology in the National Institute of Oncology, Budapest, Hungary.

*siRNA knockdown experiments*

Cells that reached 70-80% confluency were trypsinized and counted using Luna II Automated Cell Counter. Cells were plated in 2500 and 7500 cells/well density (for non-targeting siRNA and HRAS siRNA treatment, respectively) on a 48well plate supplemented with pre-mixed Lipofectamine RNAIMAX Reagent (Thermo Fisher) and OptiMEM (Thermo Fisher) containing 2.5 pmol/well non-targeting or HRAS siRNA according to the manufacturer's instruction (reverse transfection protocol). Next day, cells were treated with tipifarnib or sotorasib with a 0, 1, 5, 25, 125 and 625 nM final concentration. On the 3rd day of the treatment, cells were supplemented with fresh pre-mixed 2.5 pmol/well siRNA diluted in Lipofectamine RNAiMAX Reagent and Optimem according to the manufacturer's instruction. After 6 days, wells were washed with DPBS (Lonza) and the cells were fixed by 10% trichloroacetic acid and stained with Sulphorodamine-B (SRB) (Sigma) dye for 15 minutes. Plates then were repeatedly washed with 1% acetic acid to remove excess dye. Protein-bound SRB was then dissolved in 10 mM Tris buffer (pH=7,4) and OD was measured at 570 nm using a microplate reader (EL800, BioTec Instruments, Winooski, VT). OD values were normalized to control, and IC50 values were calculated from transformed data using GraphPad PRISM 5 software.

Example 1. 2D tumor cell culture combinational tests using KRAS mutant specific inhibitors and FTIs

Tables 3. Growth inhibition assay with single agent or combination of AMG510 (sotorasib) and tipifarnib. (A1-A9) Cell lines were treated with different concentrations of AMG510 or tipifarnib or both for six days. Relative cell viability are shown (mean, SD). (B1-B9) Combination indexes of AMG510 and tipifarnib combinational treatment. Combination indexes (CI) were calculated by CompuSyn Software from the data of viability assays (A) of AMG510 and tipifarnib combination treatment. CI values less than 1 indicate synergy while values equal to or more than 1 represent additive or antagonistic effect, respectively.

3A/1

| H358 | 0 nM AMG510 | | 25 nM AMG510 | | 50 nM AMG510 | | 100 nM AMG510 | |
|---|---|---|---|---|---|---|---|---|
| | MEAN | SD | MEAN | SD | MEAN | SD | MEAN | SD |
| 0 $\mu$M tipifarnib | 1.000000 | 0.000000 | 0.3613376 | 0.09168658 | 0.178872 | 0.02804614 | 0.1052535 | 0.00884063 |
| 1 $\mu$M tipifarnib | 0.3971009 | 0.08712061 | 0.1390393 | 0.05524015 | 0.08304357 | 0.03075656 | 0.06117455 | 0.02035107 |
| 2 $\mu$M tipifarnib | 0.2601672 | 0.1333925 | 0.08710403 | 0.05471783 | 0.06827532 | 0.03377457 | 0.06205897 | 0.03134832 |
| 4 $\mu$M tipifarnib | 0.2080783 | 0.1169764 | 0.06853954 | 0.05426301 | 0.06936473 | 0.04571458 | 0.05129578 | 0.02557168 |
| n=3 | | | | | | | | |

3A2

| H358 | 0 nM AMG510 | | | 10 nM AMG510 | | | 25 nM AMG510 | | | 50 nM AMG510 | | |
|---|---|---|---|---|---|---|---|---|---|---|---|---|
| | MEAN | SD | N | MEAN | SD | N | MEAN | SD | N | MEAN | SD | N |
| 0 nM tipifarnib | 1.000 | 0.000 | 3 | 0.596 | 0.044 | 3 | 0.345 | 0.029 | 3 | 0.204 | 0.043 | 3 |
| 50 nM tipifarnib | 0,029 | 0.038 | 3 | 0.467 | 0.034 | 3 | 0.324 | 0.013 | 3 | 0.189 | 0.007 | 3 |
| 100 nM tipifarnib | 0.737 | 0.082 | 3 | 0.432 | 0.029 | 3 | 0.291 | 0.020 | 3 | 0.183 | 0.011 | 3 |
| 500 nM tipifarnib | 0.608 | 0.017 | 2 | 0.312 | 0.042 | 2 | 0.214 | 0.015 | 2 | 0.137 | 0.022 | 2 |

3A/3

| H1792 | 0 nM AMG510 | | 25 nM AMG510 | | 50 nM AMG510 | | 100 nM AMG510 | | |
|---|---|---|---|---|---|---|---|---|---|
| | MEAN | SD | MEAN | SD | MEAN | SD | MEAN | SD | N |
| 0 μM TIPIFARNIB | 1.000 | 0.000 | 0.786 | 0.049 | 0.639 | 0.011 | 0.682 | 0.049 | 2 |
| 1 μM TIPIFARNIB | 0.200 | 0.029 | 0.150 | 0.000 | 0.103 | 0.021 | 0.108 | 0.021 | 2 |
| 2 μM TIPIFARNIB | 0.155 | 0.006 | 0.111 | 0.009 | 0.112 | 0.030 | 0.114 | 0.044 | 2 |
| 4 μM TIPIFARNIB | 0.144 | 0.045 | 0.098 | 0.017 | 0.102 | 0.016 | 0.089 | 0.012 | 2 |

3A/4

| SW1573 | 0 nM AMG510 | | 25 nM AMG510 | | 50 nM AMG510 | | 100 nM AMG510 | |
|---|---|---|---|---|---|---|---|---|
| | MEAN | SD | MEAN | SD | MEAN | SD | MEAN | SD |
| 0 | 1.000000 | 0.000000 | 0.9447713 | 0.03872463 | 0.9193989 | 0.07219566 | 0.8979381 | 0.1037513 |
| 1 μM tipi-farnib | 0.3533176 | 0.1373859 | 0.2704512 | 0.1444721 | 0.2545759 | 0.127141 | 0.2510503 | 0.1510134 |
| 2 μM tipi-farnib | 0.3122584 | 0.1998727 | 0.2354344 | 0.152202 | 0.2295205 | 0.1368567 | 0.2105447 | 0.1528429 |
| 4 μM tipi-farnib | 0.2632048 | 0.1989226 | 0.1905316 | 0.1506057 | 0.1894055 | 0.1252749 | 0.1835937 | 0.1314849 |
| n=2 | | | | | | | | |

3A/5

| SW1573 | 0 nM AMG510 | | 50 nM AMG510 | | 100 nM AMG510 | | 500 nM AMG510 | | |
|---|---|---|---|---|---|---|---|---|---|
| | MEAN | SD | MEAN | SD | MEAN | SD | MEAN | SD | N |
| 0 nM tipifarnib | 1.000 | 0.000 | 0.898 | 0.046 | 0.873 | 0.029 | 0.773 | 0.061 | 3 |
| 10 nM tipifarnib | 0.672 | 0.039 | 0.399 | 0.056 | 0.379 | 0.048 | 0.333 | 0.051 | 3 |
| 25 nM tipifarnib | 0.518 | 0.042 | 0.308 | 0.043 | 0.294 | 0.038 | 0.269 | 0.056 | 3 |
| 50 nM tipifarnib | 0.425 | 0.027 | 0.263 | 0.031 | 0.246 | 0.032 | 0.226 | 0.039 | 3 |

3A/6

| PF139 | 0 nM AMG510 | | 25 nM AMG510 | | 50 nM AMG510 | | 100 nM AMG510 | |
|---|---|---|---|---|---|---|---|---|
| | MEAN | SD | MEAN | SD | MEAN | SD | MEAN | SD |
| 0 | 1.000000 | 0.000000 | 0.7179138 | 0.01447082 | 0.6271546 | 0.08527465 | 0.5512518 | 0.06393141 |
| 1 $\mu$M tipifarnib | 0.3825193 | 0.01476359 | 0.2951131 | 0.0321155 | 0.2611449 | 0.00821747 | 0.2314856 | 0.0205883 |
| 2 $\mu$M tipifarnib | 0.3509771 | 0.000529261 | 0.2479616 | 0.007678545 | 0.218426 | 0.01268066 | 0.2106754 | 0.02284235 |
| 4 $\mu$M tipifarnib | 0.2341527 | 0.01681639 | 0.1857185 | 0.01017932 | 0.1802351 | 0.050705 | 0.1468956 | 0.03071361 |
| n=2 | | | | | | | | |

3A/7

| PF139 | 0 nM AMG510 | | 50 nM AMG510 | | 100 nM AMG510 | | 500 nM AMG510 | | |
|---|---|---|---|---|---|---|---|---|---|
| | MEAN | SD | MEAN | SD | MEAN | SD | MEAN | SD | N |
| 0 nM tipifarnib | 1.000 | 0.000 | 0.843 | 0.025 | 0.809 | 0.042 | 0.727 | 0.009 | 4 |
| 100 nM tipifarnib | 0.835 | 0.102 | 0.772 | 0.171 | 0.686 | 0.117 | 0.623 | 0.128 | 4 |
| 500 nM tipifarnib | 0.639 | 0.071 | 0.540 | 0.073 | 0.529 | 0.064 | 0.479 | 0.082 | 4 |
| 1000 nM tipifarnib | 0.526 | 0.011 | 0.470 | 0.039 | 0.481 | 0.042 | 0.444 | 0.044 | 3 |

3A/8

| PF97 | 0 nM AMG510 | | 50 nM AMG510 | | 100 nM AMG510 | | 500 nM AMG510 | |
|---|---|---|---|---|---|---|---|---|
| | MEAN | SD | MEAN | SD | MEAN | SD | MEAN | SD |
| 0 | 1.000000 | 0.000000 | 0.4171837 | 0.1457587 | 0.3581596 | 0.1285327 | 0.360164 | 0.1461214 |
| 100 nM tipifarnib | 0.7714267 | 0.09639794 | 0.391387 | 0.1138881 | 0.3379096 | 0.07608321 | 0.3052404 | 0.1011889 |
| 500 nM tipifarnib | 0.6250134 | 0.07620144 | 0.337027 | 0.08006021 | 0.3050631 | 0.07917152 | 0.2958313 | 0.0892304 |
| 1000 nM tipifarnib | 0.6169425 | 0.07660785 | 0.3122901 | 0.06917624 | 0.280222 | 0.08603458 | 0.2720837 | 0.06121228 |
| n=3 | | | | | | | | |

3A/9

| MIAPACA2 | 0 nM AMG510 | | 25 nM AMG510 | | 50 nM AMG510 | | 100 nM AMG510 | | N |
|---|---|---|---|---|---|---|---|---|---|
| | MEAN | SD | MEAN | SD | MEAN | SD | MEAN | SD | |
| 0 nM tipifarnib | 1 | 0 | 0.7475432 | 0.1613564 | 0.5812865 | 0.1536619 | 0.4368667 | 0.1130516 | 3 |
| 5 nM tipifarnib | 0.5278819 | 0.1920417 | 0.3848505 | 0.1773259 | 0.2968681 | 0.1242116 | 0.2142874 | 0.0738077 | 3 |
| 10 nM tipifarnib | 0.3776034 | 0.129293 | 0.2503251 | 0.097962 | 0.2058956 | 0.0670527 | 0.1617614 | 0.0401955 | 3 |
| 25 nM tipifarnib | 0.2605363 | 0.0605214 | 0.1653899 | 0.0586967 | 0.1519774 | 0.0398397 | 0.1339166 | 0.0268909 | 3 |

3B/1

| H358 | 25 nM AMG510 | 50 nM AMG510 | 100 nM AMG510 |
|---|---|---|---|
| 1 μM tipifarnib | 0.47849 | 0.47223 | 0.656560 |
| 2 μM tipifarnib | 0.33645 | 0.42419 | 0.700990 |
| 4 μM tipifarnib | 0.33652 | 0.51160 | 0.626860 |

3B/2

| H358 | 10 nM AMG510 | 25 nM AMG510 | 50 nM AMG510 |
|---|---|---|---|
| 50 nM tipifarnib | 0.645 | 0.902 | 0.928 |
| 100 nM tipifarnib | 0.575 | 0.784 | 0.895 |
| 500 nM tipifarnib | 0.371 | 0.542 | 0.653 |

3B/3

| H1792 | 25 nM AMG510 | 50 nM AMG510 | 100 nM AMG510 |
|---|---|---|---|
| 1 μM tipifarnib | 0.33724 | 0.07523 | 0.090980 |
| 2 μM tipifarnib | 0.20089 | 0.20836 | 0.223950 |
| 4 μM tipifarnib | 0.24683 | 0.28855 | 0.170580 |

3B/4

| SW1573 | 25 nM AMG510 | 50 nM AMG510 | 100 nM AMG510 |
|---|---|---|---|
| 1 μM tipifarnib | 0.27313 | 0.21241 | 0.198840 |
| 2 μM tipifarnib | 0.29778 | 0.27198 | 0.189970 |
| 4 μM tipifarnib | 0.25206 | 0.24174 | 0.217350 |

3B/5

| SW1573 | 50 nM AMG510 | 100 nM AMG510 | 500 nM AMG510 |
|---|---|---|---|
| 10 nM tipifarnib | 0.601 | 0.621 | 0.621 |
| 25 nM tipifarnib | 0.692 | 0.706 | 0.731 |
| 50 nM tipifarnib | 0.737 | 0.754 | 0.774 |

3B/6

| PF139 | 25 nM AMG510 | 50 nM AMG510 | 100 nM AMG510 |
|---|---|---|---|
| 1 μM tipifarnib | 0.43502 | 0.33673 | 0.281310 |
| 2 μM tipifarnib | 0.52551 | 0.39395 | 0.391850 |
| 4 μM tipifarnib | 0.50277 | 0.47466 | 0.308310 |

3B/7

| PF139 | 50 nM AMG510 | 100 nM AMG510 | 500 nM AMG510 |
|---|---|---|---|
| 100 nM tipifarnib | 0.767 | 0.384 | 0.391 |
| 500 nM tipifarnib | 0.548 | 0.517 | 0.403 |
| 1000 nM tipifarnib | 0.709 | 0.761 | 0.621 |

3B/8

| PF97 | 50 nM AMG510 | 100 nM AMG510 | 500 nM AMG510 |
|---|---|---|---|
| 100 nM tipifarnib | 0.712630 | 0.099490 | 0.082960 |
| 500 nM tipifarnib | 0.066730 | 0.030070 | 0.060820 |
| 1000 nM tipifarnib | 0.042700 | 0.023560 | 0.029580 |

3B/9

| MIAPACA2 | 25 nM AMG510 | 50 nM AMG510 | 100 nM AMG510 |
|---|---|---|---|
| 5 nM tipifarnib | 0.675 | 0.545 | 0.494 |
| 10 nM tipifarnib | 0.494 | 0.437 | 0.424 |
| 25 nM tipifarnib | 0.530 | 0.516 | 0.522 |

Tables 4. Growth inhibition assay with single agent or combination of AMG510 (sotorasib) and lonafarnib. (A1-A4) Cell lines were treated with different concentrations of AMG510 or lonafarnib or both for six days. Relative cell viability are shown (mean, SD). (B1-B4) Combination indexes of AMG510 and lonafarnib combinational treatment. Combination indexes (CI) were calculated by CompuSyn Software from the data of viability assays (A) of AMG510 and lonafarnib combination treatment. CI values less than 1 indicate synergy while values equal to or more than 1 represent additive or antagonistic effect, respectively.

4/A1

| H358 | 0 nM AMG510 | | 25 nM AMG510 | | 50 nM AMG510 | | 100 nM AMG510 | | |
|---|---|---|---|---|---|---|---|---|---|
| | MEAN | SD | MEAN | SD | MEAN | SD | MEAN | SD | N |
| 0 nM lonafarnib | 1.000 | 0.000 | 0.275 | 0.035 | 0.150 | 0.016 | 0.073 | 0.007 | 3 |
| 250 nM lonafarnib | 0.571 | 0.090 | 0.195 | 0.034 | 0.116 | 0.011 | 0.060 | 0.000 | 3 |
| 500 nM lonafarnib | 0.503 | 0.085 | 0.179 | 0.023 | 0.109 | 0.007 | 0.062 | 0.008 | 3 |
| 1000 nM lonafarnib | 0.402 | 0.045 | 0.147 | 0.003 | 0.090 | 0.004 | 0.053 | 0.018 | 3 |

4/A2

| H1792 | 0 nM AMG510 | | 25 nM AMG510 | | 50 nM AMG510 | | 100 nM AMG510 | | |
|---|---|---|---|---|---|---|---|---|---|
| | MEAN | SD | MEAN | SD | MEAN | SD | MEAN | SD | N |
| 0 nM lonafarnib | 1.000 | 0.000 | 0.759 | 0.042 | 0.721 | 0.062 | 0.693 | 0.086 | 3 |
| 250 nM lonafarnib | 0.237 | 0.034 | 0.216 | 0.024 | 0.212 | 0.024 | 0.206 | 0.022 | 3 |
| 500 nM lonafarnib | 0.185 | 0.025 | 0.172 | 0.022 | 0.176 | 0.027 | 0.163 | 0.029 | 3 |
| 1000 nM lonafarnib | 0.151 | 0.035 | 0.129 | 0.015 | 0.118 | 0.011 | 0.115 | 0.017 | 3 |

4/A3

| PF139 | 0 nM AMG510 | | 25 nM AMG510 | | 50 nM AMG510 | | 100 nM AMG510 | | |
|---|---|---|---|---|---|---|---|---|---|
| | MEAN | SD | MEAN | SD | MEAN | SD | MEAN | SD | N |
| 0 nM lonafarnib | 1.000 | 0.000 | 0.730 | 0.066 | 0.624 | 0.042 | 0.564 | 0.032 | 3 |
| 250 nM lonafarnib | 0.917 | 0.142 | 0.600 | 0.098 | 0.508 | 0.076 | 0.489 | 0.045 | 3 |
| 500 nM lonafarnib | 0.884 | 0.036 | 0.535 | 0.069 | 0.441 | 0.076 | 0.424 | 0.062 | 3 |
| 1000 nM lonafarnib | 0.649 | 0.110 | 0.439 | 0.076 | 0.366 | 0.058 | 0.354 | 0.067 | 3 |

4/A4

| SW1573 | 0 nM AMG510 | | 25 nM AMG510 | | 50 nM AMG510 | | 100 nM AMG510 | | |
|---|---|---|---|---|---|---|---|---|---|
| | MEAN | SD | MEAN | SD | MEAN | SD | MEAN | SD | N |
| 0 nM lonafarnib | 1.000 | 0.000 | 0.875 | 0.061 | 0.862 | 0.098 | 0.857 | 0.055 | 3 |
| 250 nM lonafarnib | 0.560 | 0.081 | 0.359 | 0.081 | 0.346 | 0.073 | 0.352 | 0.072 | 3 |
| 500 nM lonafarnib | 0.503 | 0.083 | 0.307 | 0.073 | 0.289 | 0.069 | 0.293 | 0.072 | 3 |
| 1000 nM lonafarnib | 0.432 | 0.089 | 0.272 | 0.067 | 0.281 | 0.057 | 0.264 | 0.060 | 3 |

4/B1

| H358 | 10 nM AMG510 | 25 nM AMG510 | 50 nM AMG510 |
|---|---|---|---|
| 250 nM lonafarnib | 0.699 | 0.788 | 0.828 |
| 500 nM lonafarnib | 0.659 | 0.748 | 0.856 |
| 1000 nM lonafarnib | 0.556 | 0.627 | 0.742 |

4/B2

| H1792 | 50 nM AMG510 | 100 nM AMG510 | 500 nM AMG510 |
|---|---|---|---|
| 250 nM lonafarnib | 0.764 | 0.720 | 0.658 |
| 500 nM lonafarnib | 0.757 | 0.811 | 0.645 |
| 1000 nM lonafarnib | 0.653 | 0.507 | 0.472 |

4/B3

| PF139 | 50 nM AMG510 | 100 nM AMG510 | 500 nM AMG510 |
|---|---|---|---|
| 250 nM LONAFARNIB | 0.544 | 0.494 | 0.745 |
| 500 nM LONAFARNIB | 0.523 | 0.443 | 0.592 |
| 1000 nM LONAFARNIB | 0.563 | 0.482 | 0.564 |

4/B4

| SW1573 | 50 nM AMG510 | 100 nM AMG510 | 500 nM AMG510 |
|---|---|---|---|
| 250 nM LONAFARNIB | 0.107 | 0.092 | 0.098 |
| 500 nM LONAFARNIB | 0.114 | 0.090 | 0.095 |
| 1000 nM LONAFARNIB | 0.144 | 0.162 | 0.129 |

Tables 5. Growth inhibition assay with single agent or combination of ARS1620 and tipifarnib. (A1-A4) Cell lines were treated with different concentrations of ARS1620 or tipifarnib or both for six days. Relative cell viability are shown (mean, SD). (B1-B4) Combination indexes of ARS1620 and tipifarnib combinational treatment. Combination indexes (CI) were calculated by CompuSyn Software from the data of viability assays (A) of ARS1620 and tipifarnib combination treatment. CI values less than 1 indicate synergy while values equal to or more than 1 represent additive or antagonistic effect, respectively.
5/A1

| H358 | 0 µM ARS1620 | | 2.5 µM ARS1620 | | 5 µM ARS1620 | | 10 µM ARS1620 | | |
|---|---|---|---|---|---|---|---|---|---|
| | MEAN | SD | MEAN | SD | MEAN | SD | MEAN | SD | N |
| 0 nM tipifarnib | 1 | 0 | 0.4003417 | 0.0741194 | 0.2175323 | 0.0587533 | 0.086745 | 0.0156189 | 3 |
| 100 nM tipifarnib | 0.6058348 | 0.0634492 | 0.2733049 | 0.0366566 | 0.1565172 | 0.0422865 | 0.0647846 | 0.0191113 | 3 |
| 500 nM tipifarnib | 0.4972108 | 0.0470536 | 0.2171875 | 0.0329644 | 0.1276544 | 0.0307267 | 0.0509767 | 0.006453 | 3 |
| 1000 nM tipifarnib | 0.4017152 | 0.0320396 | 0.1688179 | 0.0212847 | 0.1013975 | 0.0223544 | 0.039116 | 0.0111307 | 3 |

5/A2

| H1792 | 0 μM ARS1620 | | 2.5 μM ARS1620 | | 5 μM ARS1620 | | 10 μM ARS1620 | | |
|---|---|---|---|---|---|---|---|---|---|
| | MEAN | SD | MEAN | SD | MEAN | SD | MEAN | SD | N |
| 0 nM tipifarnib | 1 | 0 | 0.7774865 | 0.0715778 | 05768189 | 0.0366529 | 02872802 | 0.0437716 | 3 |
| 100 nM tipifarnib | 0.2742055 | 0.1318137 | 0.2149945 | 0.0750434 | 0.1774695 | 0.0619017 | 0.1017871 | 0.0413067 | 3 |
| 500 nM tipifarnib | 0.1645074 | 0.0723114 | 0.1115123 | 0.034041 | 0.0988723 | 0.0355874 | 0.0753453 | 0.0467295 | 3 |
| 1000 nM tipifarnib | 0.1291366 | 0.0597371 | 0.0943531 | 0.0552164 | 0.0742812 | 0.042767 | 0.0595698 | 0.0462845 | 3 |

5/A3

| PF139 | 0 μM ARS1620 | | 2.5 μM ARS1620 | | 5 μM ARS1620 | | 10 μM ARS1620 | | |
|---|---|---|---|---|---|---|---|---|---|
| | MEAN | SD | MEAN | SD | MEAN | SD | MEAN | SD | N |
| 0 nM tipifarnib | 1 | 0 | 0.5320728 | 0.0439921 | 0.4321444 | 0.0227727 | 0.308686 | 0.0332933 | 3 |
| 100 nM tipifarnib | 0.7376069 | 0.1017608 | 0.3713624 | 0.057955 | 0.2642342 | 0.0591497 | 0.1875982 | 0.0142996 | 3 |
| 500 nM tipifarnib | 0.5232075 | 0.0622653 | 0.2488282 | 0.0295014 | 0.2031557 | 0.0274619 | 0.1205219 | 0.0316906 | 3 |
| 1000 nM tipifarnib | 0.3993895 | 0.093512 | 0.1882719 | 0.0448345 | 0.1689706 | 0.029084 | 0.1151869 | 0.0271503 | 3 |

EP 4 426 303 B1

5/A4

| SW1573 | 0 μM ARS1620 | | 2.5 μM ARS1620 | | 5 μM ARS1620 | | 10 μM ARS1620 | | |
|---|---|---|---|---|---|---|---|---|---|
| | MEAN | SD | MEAN | SD | MEAN | SD | MEAN | SD | N |
| 0 nM tipifarnib | 1 | 0 | 0.9293731 | 0.0159791 | 0.9012464 | 0.0544454 | 0.8380404 | 0.1215456 | 2 |
| 100 nM tipifarnib | 0.3698213 | 0.0416959 | 0.2468764 | 0.0333408 | 0.2192609 | 0.0409316 | 0.1950268 | 0.0502953 | 2 |
| 500 nM tipifarnib | 0.249589 | 0.0386815 | 0.1646621 | 0.0329327 | 0.1614702 | 0.0374467 | 0.1371835 | 0.0377079 | 2 |
| 1000 nM tipifarnib | 0.2122399 | 0.0325072 | 0.1624657 | 0.0373499 | 0.1465379 | 0.0388995 | 0.135003 | 0.0316148 | 2 |

5/B1

| H358 | 2.5 μM ARS1620 | 5 μM ARS1620 | 10 μM ARS1620 |
|---|---|---|---|
| 100 nM tipifarnib | 0.664 | 0.789 | 0.780 |
| 500 nM tipifarnib | 0.554 | 0.669 | 0.649 |
| 1000 nM tipifarnib | 0.445 | 0.554 | 0.532 |

5/B2

| H1792 | 2.5 μM ARS1620 | 5 μM ARS1620 | 10 μM ARS1620 |
|---|---|---|---|
| 100 nM tipifarnib | 0.642 | 0.573 | 0.482 |
| 500 nM tipifarnib | 0.449 | 0.448 | 0.459 |
| 1000 nM tipifarnib | 0.514 | 0.388 | 0.422 |

5/B3

| PF139 | 2.5 μM ARS1620 | 5 μM ARS1620 | 10 μM ARS1620 |
|---|---|---|---|
| 100 nM tipifarnib | 0.444 | 0.384 | 0.382 |
| 500 nM tipifarnib | 0.311 | 0.312 | 0.206 |
| 1000 nM tipifarnib | 0.266 | 0.292 | 0.223 |

5/B4

| SW1573 | 2.5 μM ARS1620 | 5 μM ARS1620 | 10 μM ARS1620 |
|---|---|---|---|
| 100 nM tipifarnib | 0.187 | 0.122 | 0.085 |
| 500 nM tipifarnib | 0.210 | 0.194 | 0.115 |
| 1000 nM tipifarnib | 0.392 | 0.282 | 0.213 |

Tables 6. Growth inhibition assay with single agent or combination of adagrasib (MRTX849) and tipifarnib. (A1-A4) Cell lines were treated with different concentrations of adagrasib or tipifarnib or both for six days. Relative cell viability are shown (mean, SD). (B1-B4) Combination indexes of adagrasib and tipifarnib combinational treatment. Combination indexes (CI) were calculated by CompuSyn Software from the data of viability assays (A) of adagrasib and tipifarnib combination treatment. CI values less than 1 indicate synergy while values equal to or more than 1 represent additive or antagonistic effect, respectively.

6/A1

| PF97 | 0 nM adagrasib | | 25 nM adagrasib | | 100 nM adagrasib | | 500 nM adagrasib | | |
|---|---|---|---|---|---|---|---|---|---|
| | MEAN | SD | MEAN | SD | MEAN | SD | MEAN | SD | N |
| 0 nM tipifarnib | 1 | 0 | 0.5819803 | 0.1414413 | 0.4670708 | 0.1593345 | 0.4587282 | 0.0962397 | 3 |
| 100 nM tipifarnib | 0.8460492 | 0.0770208 | 0.493707 | 0.1075403 | 0.3751976 | 0.1226322 | 0.3752247 | 0.1289255 | 3 |
| 500 nM tipifarnib | 0.734102 | 0.1160101 | 0.4530128 | 0.107172 | 0.3973234 | 0.0939526 | 0.3662243 | 0.0758861 | 3 |
| 1000 nM tipifarnib | 0.5824456 | 0.0324539 | 0.3869061 | 0.1189263 | 0.3326227 | 0.1080859 | 0.3099594 | 0.1089222 | 3 |

6/A2

| MIAPACA2 | 0 nM adagrasib | | 25 nM adagrasib | | 50 nM adagrasib | | 100 nM adagrasib | | |
|---|---|---|---|---|---|---|---|---|---|
| | MEAN | SD | MEAN | SD | MEAN | SD | MEAN | SD | N |
| 0 nM tipifarnib | 1 | 0 | 0.5044294 | 0.1666235 | 0.3826428 | 0.1488887 | 0.2894268 | 0.0954429 | 3 |
| 5 nM tipifarnib | 0.5026853 | 0.1772494 | 0.2511497 | 0.1224021 | 0.2035188 | 0.0842621 | 0.1719743 | 0.0586717 | 3 |
| 10 nM tipifarnib | 0.3711883 | 0.1028751 | 0.1721051 | 0.0509687 | 0.1690798 | 0.0634522 | 0.1461318 | 0.0461358 | 3 |
| 25 nM tipifarnib | 0.2459463 | 0.0664656 | 0.1449029 | 0.0546994 | 0.1393232 | 0.0490333 | 0.1323381 | 0.044648 | 3 |

6/A3

| SW1573 | 0 nM ADAGRASIB | | 10 nM ADAGRASIB | | 25 nM ADAGRASIB | | 100 nM ADAGRASIB | | |
|---|---|---|---|---|---|---|---|---|---|
| | MEAN | SD | MEAN | SD | MEAN | SD | MEAN | SD | N |
| 0 nM tipifarnib | 1 | 0 | 0.8526796 | 0.0553104 | 0.7729907 | 0.0637624 | 0.6716625 | 0.0387517 | 3 |
| 50 nM tipifarnib | 0.4842397 | 0.0056923 | 0.2863175 | 0.0230686 | 0.2566222 | 0.0194419 | 0.2576646 | 0.0195566 | 3 |
| 250 nM tipifarnib | 0.321648 | 0.0183179 | 0.2170887 | 0.0268994 | 0.1951117 | 0.0169398 | 0.1900459 | 0.0203644 | 3 |
| 500 nM tipifarnib | 0.2685286 | 0.0139627 | 0.1915538 | 0.0163917 | 0.1765996 | 0.004218 | 0.1691722 | 0.0179545 | 3 |

6/A4

| PF139 | 0 nM ADAGRASIB | | 10 nM ADAGRASIB | | 25 nM ADAGRASIB | | 100 nM ADAGRASIB | | |
|---|---|---|---|---|---|---|---|---|---|
| | MEAN | SD | MEAN | SD | MEAN | SD | MEAN | SD | N |
| 0 nM tipifarnib | 1 | 0 | 0.7257477 | 0.0356981 | 0.666486 | 0.0686037 | 0.6476439 | 0.0729372 | 3 |
| 50 nM tipifarnib | 0.846766 | 0.0386377 | 0.6237329 | 0.0685298 | 0.5387785 | 0.0398686 | 0.4844255 | 0.0563187 | 3 |
| 250 nM tipifarnib | 0.5738306 | 0.0659694 | 0.4332412 | 0.0505521 | 0.3910596 | 0.0552102 | 0.3498146 | 0.0285874 | 3 |
| 500 nM tipifarnib | 0.4319484 | 0.0024183 | 0.3634072 | 0.0556947 | 0.3163726 | 0.0355307 | 0.2858451 | 0.0371029 | 3 |

6/B1

| PF97 | ADAGRASIB 25 nM | ADAGRASIB 100 nM | ADAGRASIB 500 nM |
|---|---|---|---|
| 100 nM TIPIFARNIB | 0.230 | 0.055 | 0.201 |
| 500 nM TIPIFARNIB | 0.229 | 0.173 | 0.229 |
| 1000 nM TIPIFARNIB | 0.213 | 0.144 | 0.140 |

6/B2

| MIAPACA2 | ADAGRASIB 25 nM | ADAGRASIB 50 nM | ADAGRASIB 100 nM |
|---|---|---|---|
| 5 nM TIPIFARNIB | 0.402 | 0.398 | 0.476 |
| 10 nM TIPIFARNIB | 0.307 | 0.387 | 0.437 |
| 25 nM TIPIFARNIB | 0.469 | 0.500 | 0.573 |

6/B3

| SW1573 | ADAGRASIB 10 nM | ADAGRASIB 25 nM | ADAGRASIB 100 nM |
|---|---|---|---|
| 50 nM TIPIFARNIB | 0.129 | 0.093 | 0.110 |
| 250 nM TIPIFARNIB | 0.259 | 0.187 | 0.179 |
| 500 nM TIPIFARNIB | 0.354 | 0.279 | 0.248 |

6/B4

| PF139 | ADAGRASIB 10 nM | ADAGRASIB 25 nM | ADAGRASIB 100 nM |
|---|---|---|---|
| 50 nM TIPIFARNIB | 0.314 | 0.182 | 0.143 |
| 250 nM TIPIFARNIB | 0.509 | 0.417 | 0.340 |
| 500 nM TIPIFARNIB | 0.726 | 0.569 | 0.483 |

Tables 7. Growth inhibition assay with single agent or combination of adagrasib (MRTX849) and lonafarnib. (A1-A3) Cell lines were treated with different concentrations of adagrasib or lonafarnib or both for six days. Relative cell viability are shown (mean, SD). (B1-B3) Combination indexes of adagrasib and lonafarnib combinational treatment. Combination indexes (CI) were calculated by CompuSyn Software from the data of viability assays (A) of adagrasib and lonafarnib combination treatment. CI values less than 1 indicate synergy while values equal to or more than 1 represent additive or antagonistic effect, respectively.

7/A1

| PF97 | 0 nM ADAGRASIB | | 10 nM ADAGRASIB | | 100 nM ADAGRASIB | | 500 nM ADAGRASIB | |
|---|---|---|---|---|---|---|---|---|
| | MEAN | SD | MEAN | SD | MEAN | SD | MEAN | SD N |
| 0 nM lonafarnib | 1 | 0 | 0.5253549 | 0.0586294 | 0.2918274 | 0.042096 | 0.1940481 | 0.0401666 3 |
| 250 nM lonafarnib | 0.7258881 | 0.0188189 | 0.3338418 | 0.0584889 | 0.1639796 | 0.027066 | 0.110369 | 0.0255196 3 |
| 500 nM lonafarnib | 0.6124089 | 0.0366064 | 0.2562615 | 0.0455159 | 0.1231886 | 0.0224446 | 0.0856813 | 0.0201734 3 |
| 1000 nM lonafarnib | 0.5110087 | 0.0490681 | 0.2127048 | 0.0390267 | 0.1100399 | 0.0322375 | 0.0758577 | 0.0121285 3 |

7/A2

| MIAPACA2 | 0 nM ADAGRASIB | | 25 nM ADAGRASIB | | 50 nM ADAGRASIB | | 100 nM ADAGRASIB | |
|---|---|---|---|---|---|---|---|---|
| | MEAN | SD | MEAN | SD | MEAN | SD | MEAN | SD N |
| 0 nM lonafarnib | 1 | 0 | 0.5078382 | 0. 1561277 | 0.3752438 | 0.1070537 | 0.2732855 | 0.0610314 3 |
| 25 nM lonafarnib | 0.9466388 | 0.027585 | 0.435186 | 0.0901005 | 0.3207552 | 0.0677755 | 0.2315796 | 0.0449655 3 |
| 50 nM lonafarnib | 0.809925 | 0.0600329 | 0.3466733 | 0.0761181 | 0.2482984 | 0.0601315 | 0.195955 | 0.0443322 3 |
| 100 nM lonafarnib | 0.4462046 | 0.069187 | 0.2296997 | 0.0569237 | 0.1895404 | 0.0319975 | 0.157195 | 0.0336202 3 |

7/A3

| SW1573 | 0 nM ADAGRASIB | | 10 nM ADAGRASIB | | 25 nM ADAGRASIB | | 100 nM ADAGRASIB | |
|---|---|---|---|---|---|---|---|---|
| | MEAN | SD | MEAN | SD | MEAN | SD | MEAN | SD N |
| 0 nM lonafarnib | 1 | 0 | 0.8657386 | 0.0505857 | 0.7939022 | 0.0541468 | 0.7249506 | 0.0519773 3 |
| 50 nM lonafarnib | 0.9220966 | 0.0173545 | 0.6243386 | 0.0313033 | 0.5355726 | 0.0387003 | 0.5088461 | 0.0353997 3 |
| 250 nM lonafarnib | 0.5821082 | 0.0241918 | 0.3701077 | 0.0306186 | 0.3323648 | 0.0349432 | 0.315332 | 0.04011 3 |
| 500 nM lonafarnib | 0.4452294 | 0.0508776 | 0.3191141 | 0.0209526 | 0.2940856 | 0.0171615 | 0.2752341 | 0.025867 3 |

7/B1

| PF97 | ADAGRASIB 10 nM | ADAGRASIB 100 nM | ADAGRASIB 500 nM |
|---|---|---|---|
| 250 nM LONAFARNIB | 0.228 | 0.151 | 0.210 |
| 500 nM LONAFARNIB | 0.152 | 0.081 | 0.114 |
| 1000 nM LONAFARNIB | 0.167 | 0.083 | 0.094 |

7/B2

| MIAPACA2 | ADAGRASIB 25 nM | ADAGRASIB 50 nM | ADAGRASIB 100 nM |
|---|---|---|---|
| 25 nM LONAFARNIB | 0.921 | 0.891 | 0.913 |
| 50 nM LONAFARNIB | 0.817 | 0.755 | 0.851 |
| 100 nM LONAFARNIB | 0.817 | 0.833 | 0.900 |

7/B3

| SW1573 | ADAGRASIB 10 nM | ADAGRASIB 25 nM | ADAGRASIB 100 nM |
|---|---|---|---|
| 50 nM LONAFARNIB | 0.235 | 0.181 | 0.232 |
| 250 nM LONAFARNIB | 0.426 | 0.372 | 0.357 |
| 500 nM LONAFARNIB | 0.703 | 0.639 | 0.595 |

Tables 8. Growth inhibition assay with single agent or combination of sotorasib (AMG510) and other FTIs. (A1-A2) Cell lines were treated with different concentrations of sotorasib or FTI277 or both; or sotorasib or BMS214662 or both for six days. Relative cell viability are shown (mean, SD). (B1-B2) Combination indexes of sotorasib and FTI277 or sotorasib and BMS214662 combinational treatment. Combination indexes (CI) were calculated by CompuSyn Software from the data of viability assays (A) of sotorasib and FTI277 and sotorasib and BMS214662 combination treatment. CI values less than 1 indicate synergy while values equal to or more than 1 represent additive or antagonistic effect, respectively.

8/A1

| SW1573 | 0 nM AMG510 | | 50 nM AMG510 | | 100 nM AMG510 | | 500 nM AMG510 | | |
|---|---|---|---|---|---|---|---|---|---|
| | MEAN | SD | MEAN | SD | MEAN | SD | MEAN | SD | N |
| 0 μM FTI277 | 1 | 0 | 0.9534262 | 0.0587536 | 0.917036 | 0.0417166 | 0.8100476 | 0.051192 | 3 |
| 2.5 μM FTI277 | 1.025663 | 0.0305269 | 0.7843204 | 0.0194366 | 0.7155558 | 0.0763811 | 0.6482229 | 0.0823764 | 3 |
| 5 μM FTI277 | 0.9522731 | 0.0225466 | 0.6296097 | 0.0982016 | 0.5848977 | 0.072863 | 0.4959279 | 0.0926821 | 3 |
| 10 μM FTI277 | 0.7285933 | 0.1092848 | 0.4633032 | 0.0796737 | 0.4364744 | 0.0819703 | 0.3710987 | 0.061861 | 3 |

8/A2

| SW1573 | 0 nM AMG510 | | 50 nM AMG510 | | 100 nM AMG510 | | 500 nM AMG510 | |
|---|---|---|---|---|---|---|---|---|
| | MEAN | SD | MEAN | SD | MEAN | SD | MEAN | SD N |
| 0 nM BMS214662 | 1 | 0 | 0.9151738 | 0.1028816 | 0.895627 | 0.1512834 | 0.8630652 | 0.1918797 3 |
| 50 nM BMS214662 | 0.8987675 | 0.0695643 | 0.6731897 | 0.2231551 | 0.6610766 | 0.2459074 | 0.6465951 | 0.3147573 3 |
| 100 nM BMS214662 | 0.7656859 | 0.1620476 | 0.5629179 | 0.2576404 | 0.5552801 | 0.2592936 | 0.5611641 | 0.3245145 3 |
| 200 nM BMS214662 | 0.3999155 | 0.3235824 | 0.3906836 | 0.317249 | 0.3754738 | 0.3134642 | 0.3643892 | 0.346622 3 |

8/B1

| SW1573 | 50 nM AMG510 | 100 nM AMG510 | 500 nM AMG510 |
|---|---|---|---|
| 2.5 μM FTI277 | 0.455 | 0.398 | 0.507 |
| 5 μM FTI277 | 0.519 | 0.497 | 0.475 |
| 10 μM FTI277 | 0.672 | 0.644 | 0.599 |

8/B2

| | 50 nM AMG510 | 100 nM AMG510 | 500 nM AMG510 |
|---|---|---|---|
| 50 nM BMS214662 | 0.434 | 0.422 | 0.412 |
| 100 nM BMS214662 | 0.675 | 0.663 | 0.673 |
| 200 nM BMS214662 | 0.930 | 0.897 | 0.874 |

Tables 9. Growth inhibition assay with single agent or combination of MRTX1133 and tipifarnib or MRTX1133 and lonafarnib. (A1-A5) Cell lines were treated with different concentrations of MRTX1133 or tipifarnib or both; or MRTX1133 or lonafarnib or both for six days. Relative cell viability are shown (mean, SD). (B1-B5) Combination indexes of MRTX1133 and tipifarnib or MRTX1133 and lonafarnib combinational treatment. Combination indexes (CI) were calculated by CompuSyn Software from the data of viability assays (A) of MRTX1133 and tipifarnib and MRTX1133 and lonafarnib combination treatment. CI values less than 1 indicate synergy while values equal to or more than 1 represent additive or antagonistic effect, respectively.
9/A1

| SW1990 | 0 nM MRTX1133 | | 2.5 nM MRTX1133 | | 5 nM MRTX1133 | | 10 nM MRTX1133 | |
|---|---|---|---|---|---|---|---|---|
| | MEAN | SD | MEAN | SD | MEAN | SD | MEAN | SD N |
| 0 nM tipifarnib | 1 | 0 | 0.7354958 | 0.0461868 | 0.5921769 | 0.0428139 | 0.4549071 | 0.0107915 3 |
| 100 nM tipifarnib | 0.8022183 | 0.0462742 | 0.5370674 | 0.0167125 | 0.4110664 | 0.0456564 | 0.3418013 | 0.0207505 3 |
| 500 nM tipifarnib | 0.702814 | 0.0578401 | 0.4083147 | 0.0352657 | 0.3181806 | 0.0299228 | 0.2711549 | 0.0294728 3 |
| 1000 nM tipifarnib | 0.6341601 | 0.0300219 | 0.3474886 | 0.0169876 | 0.2647287 | 0.0075096 | 0.2178197 | 0.0095798 3 |

9/A2

| SNUC2B | 0 nM MRTX1133 | | 50 nM MRTX1133 | | 100 nM MRTX1133 | | 500 nM MRTX1133 | | |
|---|---|---|---|---|---|---|---|---|---|
| | MEAN | SD | MEAN | SD | MEAN | SD | MEAN | SD | N |
| 0 nM tipifarnib | 1.000000 | 0.000000 | 0.738952 | 0.1364162 | 0.7023375 | 0.08703277 | 0.6566638 | 0.0140983 | 3 |
| 1 nM tipifarnib | 0.9312939 | 0.0557229 | 0.6874732 | 0.01374405 | 0.6302841 | 0.01519079 | 0.5190245 | 0.01367404 | 2 |
| 5 nM tipifarnib | 0.8077512 | 0.1247825 | 0.6099476 | 0.06109435 | 0.610353 | 0.0770993 | 0.5492977 | 0.02680767 | 3 |
| 25 nM tipifarnib | 0.7491937 | 0.03834185 | 0.5705458 | 0.01673884 | 0.5773984 | 0.04532284 | 0.5259835 | 0.03667993 | 3 |
| 125 nM tipifarnib | 0.5947266 | 0.02181336 | 0.5105407 | 0.02837813 | 0.5077521 | 0.0350695 | 0.4657486 | 0.01856166 | 3 |
| 625 nM tipifarnib | 0.5247747 | 0.06865659 | 0.444383 | 0.03957831 | 0.4353862 | 0.01623288 | 0.3981523 | 0.01813857 | 3 |

9/A3

| H838 G12D | 0 nM MRTX1133 | | 25 nM MRTX1133 | | 125 nM MRTX1133 | | 625 nM MRTX1133 | |
|---|---|---|---|---|---|---|---|---|
| | MEAN | SD | MEAN | SD | MEAN | SD | MEAN | SD N |
| 0 nM tipifarnib | 1 | 0 | 0.899687 | 0.050297 | 0.795472 | 0.08867 | 0.690874 | 0.075259 2 |
| 25 nM tipifarnib | 0.95505 | 0.08101 | 0.840936 | 0.033094 | 0.746578 | 0.048806 | 0.628888 | 0.062632 2 |
| 125 nM tipifarnib | 0.821334 | 0.034654 | 0.760262 | 0.042536 | 0.697724 | 0.04377 | 0.582946 | 0.053478 2 |
| 625 nM tipifarnib | 0.705089 | 0.037714 | 0.637662 | 0.085106 | 0.56302 | 0.055495 | 0.496049 | 0.076081 2 |

9/A4

| PF906 | 0 nM MRTX1133 | | 25 nM MRTX1133 | | 125 nM MRTX1133 | | 625 nM MRTX1133 | |
|---|---|---|---|---|---|---|---|---|
| | MEAN | SD | MEAN | SD | MEAN | SD | MEAN | SD N |
| 0 nM tipifarnib | 1.000 | 0.000 | 0.716 | 0.095 | 0.625 | 0.126 | 0.574 | 0.130 2 |
| 25 nM tipifarnib | 0.928 | 0.019 | 0.689 | 0.053 | 0.614 | 0.111 | 0.586 | 0.097 2 |
| 125 nM tipifarnib | 0.908 | 0.056 | 0.615 | 0.116 | 0.578 | 0.099 | 0.554 | 0.078 2 |
| 625 nM tipifarnib | 0.799 | 0.002 | 0.564 | 0.090 | 0.517 | 0.061 | 0.500 | 0.065 2 |

9/A5

| SW1990 | 0 nM MRTX1133 | | 2.5 nM MRTX1133 | | 5 nM MRTX1133 | | 10 nM MRTX1133 | |
|---|---|---|---|---|---|---|---|---|
| | MEAN | SD | MEAN | SD | MEAN | SD | MEAN | SD N |
| 0 nM lonafarnib | 1 | 0 | 0.8480498 | 0.2086096 | 0.700533 | 0.2263006 | 0.5542656 | 0.1952563 3 |
| 200 nM lonafarnib | 0.8311992 | 0.1092101 | 0.7350917 | 0.1916373 | 0.5749853 | 0.1828641 | 0.4628078 | 0.1616551 3 |
| 1000 nM lonafarnib | 0.6475994 | 0.0496973 | 0.5310633 | 0.1554458 | 0.4582418 | 0.1560031 | 0.3442566 | 0.1202789 3 |
| 2000 nM lonafarnib | 0.5756068 | 0.0073335 | 0.4535933 | 0.1684658 | 0.3740162 | 0.1261492 | 0.3061061 | 0.0983414 3 |

9/B1

| SW1990 | 2.5 nM MRTX1133 | 5.0 nM MRTX1133 | 10 nM MRTX1133 |
|---|---|---|---|
| 100 nM TIPIFARNIB | 0.403 | 0.422 | 0.595 |
| 500 nM TIPIFARNIB | 0.241 | 0.273 | 0.409 |
| 1000 nM TIPIFARNIB | 0.188 | 0.206 | 0.295 |

9/B2

| SNUC2B | 50 nM MRTX1133 | 100 nM MRTX1133 | 500 nM MRTX1133 |
|---|---|---|---|
| 1 nM TIPIFARNIB | 0.27679 | 0.10380 | 0.02984 |
| 5 nM TIPIFARNIB | 0.06128 | 0.08917 | 0.07595 |
| 25 nM TIPIFARNIB | 0.11739 | 0.14531 | 0.09742 |
| 125 nM TIPIFARNIB | 0.28520 | 0.28732 | 0.18221 |
| 625 nM TIPIFARNIB | 0.70088 | 0.63636 | 0.43504 |

9/B3

| H838 G12D | 25 nM MRTX1133 | 125 nM MRTX1133 | 625 nM MRTX1133 |
|---|---|---|---|
| 25 nM TIPIFARNIB | 0.48757 | 0.49317 | 0.61985 |
| 125 nM TIPIFARNIB | 0.47593 | 0.47782 | 0.49039 |
| 625 nM TIPIFARNIB | 0.81821 | 0.56184 | 0.50346 |

9/B4

| PF906 | 25 nM MRTX1133 | 125 nM MRTX1133 | 625 nM MRTX1133 |
|---|---|---|---|
| 25 nM TIPIFARNIB | 0.617 | 0.566 | 1.555 |
| 125 nM TIPIFARNIB | 0.130 | 0.271 | 0.803 |
| 625 nM TIPIFARNIB | 0.074 | 0.095 | 0.281 |

9/B5

| SW1990 | 2.5 nM MRTX1133 | 5 nM MRTX1133 | 10 nM MRTX1133 |
|---|---|---|---|
| 200 nM LONAFARNIB | 0.919 | 0.667 | 0.788 |
| 1000 nM LONAFARNIB | 0.625 | 0.594 | 0.567 |
| 2000 nM LONAFARNIB | 0.628 | 0.515 | 0.546 |

Tables 10. Growth inhibition assay with single agent or combination of a pan-KRAS inhibitor and tipifarnib or a pan-KRAS inhibitor and lonafarnib. (A1-A7) Cell lines were treated with different concentrations of the pan-KRAS inhibitor or tipifarnib or both; or the pan-KRAS inhibitor or lonafarnib or both for six days. Relative cell viability are shown (mean, SD). (B1-B7) Combination indexes of the pan-KRAS inhibitor and tipifarnib or the pan-KRAS inhibitor and lonafarnib combinational treatment. Combination indexes (CI) were calculated by CompuSyn Software from the data of viability assays (A) of the pan-KRAS inhibitor and tipifarnib and the pan-KRAS inhibitor and lonafarnib combination treatment. CI values less than 1 indicate synergy while values equal to or more than 1 represent additive or antagonistic effect, respectively.

10/A1

| SW1573 | 0 μM BI2852 | | 10 μM BI2852 | | 20 μM BI2852 | | 30 μM BI2852 | | |
|---|---|---|---|---|---|---|---|---|---|
| | MEAN | SD | MEAN | SD | MEAN | SD | MEAN | SD | N |
| 0 nM tipifarnib | 1 | 0 | 0.9141091 | 0.0269067 | 0.7793646 | 0.0343357 | 0.574425 | 0.0514565 | 3 |
| 25 nM tipifarnib | 0.4138406 | 0.0163292 | 0.3353618 | 0.0092038 | 0.2891274 | 0.0057952 | 0.2569454 | 0.0169886 | 3 |
| 50 nM tipifarnib | 0.3536577 | 0.0131662 | 0.2861403 | 0.0071933 | 0.2508902 | 0.0095601 | 0.217796 | 0.0157331 | 3 |
| 100 nM tipifarnib | 0.2921826 | 0.0037144 | 0.2445563 | 0.0107095 | 0.2251983 | 0.011853 | 0.1890143 | 0.0047707 | 3 |

10/A2

| SW1990 | 0 μM BI2852 | | 10 μM BI2852 | | 20 μM BI2852 | | 30 μM BI2852 | | |
|---|---|---|---|---|---|---|---|---|---|
| | MEAN | SD | MEAN | SD | MEAN | SD | MEAN | SD | N |
| 0 nM tipifarnib | 1 | 0 | 0.9286902 | 0.0494788 | 0.8553712 | 0.0823626 | 0.8186821 | 0.0828383 | 3 |
| 100 nM tipifarnib | 0.7558976 | 0.0630077 | 0.6799259 | 0.080028 | 0.6284118 | 0.0642699 | 0.5790097 | 0.0894388 | 3 |
| 500 nM tipifarnib | 0.6701761 | 0.0422878 | 0.5634122 | 0.0544042 | 0.5171603 | 0.0648408 | 0.4910745 | 0.0526165 | 3 |
| 1000 nM tipifarnib | 0.549878 | 0.0363064 | 0.5099778 | 0.0444798 | 0.4875131 | 0.0335422 | 0.4445686 | 0.0496178 | 3 |

10/A3

| SNUC2B | 0 µM BI2852 | | 10 µM BI2852 | | 20 µM BI2852 | | 30 µM BI2852 | | |
|---|---|---|---|---|---|---|---|---|---|
| | MEAN | SD | MEAN | SD | MEAN | SD | MEAN | SD | N |
| 0 nM tipifarnib | 1.000000 | 0.000000 | 0.8371533 | 0.09058329 | 0.747742 | 0.02295612 | 0.6639584 | 0.06537257 | 3 |
| 1 nM tipifarnib | 0.9312939 | 0.0557229 | 0.7501897 | 0.07009698 | 0.6575422 | 0.218388 | 0.5849586 | 0.1069189 | 2 |
| 5 nM tipifarnib | 0.8077512 | 0.1247825 | 0.6904492 | 0.00419463 | 0.6244431 | 0.0776407 | 0.6105596 | 0.0616699 | 3 |
| 25 nM tipifarnib | 0.7491937 | 0.03834185 | 0.6195984 | 0.0704099 | 0.6110958 | 0.06872475 | 0.5574675 | 0.05446858 | 3 |
| 125 nM tipifarnib | 0.5947266 | 0.02181336 | 0.5192361 | 0.04444894 | 0.5276606 | 0.04801955 | 0.4939899 | 0.04110833 | 3 |
| 625 nM tipifarnib | 0.5247747 | 0.06865659 | 0.4701199 | 0.04437847 | 0.4653047 | 0.04351222 | 0.4340206 | 0.05548417 | 3 |

10/A4

| SW480 | 0 μM BI2852 | | 5 μM BI2852 | | 10 μM BI2852 | | 15 μM BI2852 | | |
|---|---|---|---|---|---|---|---|---|---|
| | MEAN | SD | MEAN | SD | MEAN | SD | MEAN | SD | N |
| 0 nM tipifarnib | 1 | 0 | 0.9311964 | 0.0841384 | 0.7460557 | 0.1952584 | 0.5370712 | 0.2870714 | 3 |
| 100 nM tipifarnib | 0.571308 | 0.2345557 | 0.4843474 | 0.1791509 | 0.3621469 | 0.0869056 | 0.24335 | 0.030951 | 3 |
| 500 nM tipifarnib | 0.4317859 | 0.2096929 | 0.341225 | 0.1416608 | 0.243976 | 0.0546527 | 0.1695473 | 0.0208795 | 3 |
| 1000 nM tipifarnib | 0.3487047 | 0.1609536 | 0.2669392 | 0.091307 | 0.1961426 | 0.0278593 | 0.132126 | 0.0173183 | 3 |

10/A5

| A549 | 0 µM BI2852 | | 20 µM BI2852 | | 30 µM BI2852 | | 40 µM BI2852 | | |
|---|---|---|---|---|---|---|---|---|---|
| | MEAN | SD | MEAN | SD | MEAN | SD | MEAN | SD | N |
| 0 nM TIPIFARNIB | 1 | 0 | 0.9541374 | 0.0458832 | 0.8483576 | 0.0922825 | 0.757593 | 0.0718919 | 3 |
| 25 nM TIPIFARNIB | 0.6232471 | 0.0090703 | 0.4292262 | 0.0592019 | 0.3767641 | 0.0781639 | 0.343847 | 0.0590104 | 2 |
| 50 nM TIPIFARNIB | 0.5256857 | 0.0281727 | 0.398333 | 0.0784567 | 0.362357 | 0.0883233 | 0.3244545 | 0.0571298 | 3 |
| 100 nM TIPIFARNIB | 0.4329806 | 0.037745 | 0.3377789 | 0.0463854 | 0.3007043 | 0.0468762 | 0.2586944 | 0.0445533 | 3 |

10/A6

| HCT116 | 0 μM BI2852 | | 10 μM BI2852 | | 20 μM BI2852 | | 30 μM BI2852 | | |
|---|---|---|---|---|---|---|---|---|---|
| | MEAN | SD | MEAN | SD | MEAN | SD | MEAN | SD | N |
| 0 nM TIPIFARNIB | 1 | 0 | 0.8460391 | 0.0367865 | 0.7086447 | 0.0517516 | 0.5906996 | 0.0424099 | 3 |
| 25 nM TIPIFARNIB | 0.5804749 | 0.0272406 | 0.4872667 | 0.0243139 | 0.4481286 | 0.0237096 | 0.4139959 | 0.0440035 | 2 |
| 50 nM TIPIFARNIB | 0.5106483 | 0.0294213 | 0.4412241 | 0.068357 | 0.4097999 | 0.0363426 | 0.3899646 | 0.0440602 | 3 |
| 100 nM TIPIFARNIB | 0.4264038 | 0.0595591 | 0.3409131 | 0.0600468 | 0.3340821 | 0.0423951 | 0.2958507 | 0.03687 | 3 |

10/A7

| SW480 | 0 µM BI2852 | | 15 µM BI2852 | | 20 µM BI2852 | | 30 µM BI2852 | | |
|---|---|---|---|---|---|---|---|---|---|
| | MEAN | SD | MEAN | SD | MEAN | SD | MEAN | SD | N |
| 0 nM lonafarnib | 1 | 0 | 0.8064414 | 0.0492166 | 0.6711577 | 0.0950329 | 0.3888371 | 0.0928369 | 3 |
| 500 nM lonafarnib | 0.719569 | 0.0591292 | 0.5821077 | 0.0745923 | 0.4884357 | 0.0849602 | 0.2840857 | 0.0644324 | 3 |
| 1000 nM lonafarnib | 0.6388632 | 0.0561698 | 0.5148935 | 0.0830442 | 0.4112212 | 0.0649291 | 0.2509059 | 0.0529399 | 3 |
| 2000 nM lonafarnib | 0.5571403 | 0.0829336 | 0.4049023 | 0.071933 | 0.324977 | 0.0688389 | 0.1937193 | 0.0476227 | 3 |

10/B1

| SW1573 | 10 μM BI2852 | 20 μM BI2852 | 30 μM BI2852 |
|---|---|---|---|
| 25 nM TIPIFARNIB | 0.600 | 0.571 | 0.615 |
| 50 nM TIPIFARNIB | 0.623 | 0.590 | 0.588 |
| 100 nM TIPIFARNIB | 0.680 | 0.678 | 0.597 |

10/B2

| SW1990 | 10 μM BI2852 | 20 μM BI2852 | 30 μM BI2852 |
|---|---|---|---|
| 100 nM TIPIFARNIB | 0.493 | 0.438 | 0.420 |
| 500 nM TIPIFARNIB | 0.539 | 0.433 | 0.425 |
| 1000 nM TIPIFARNIB | 0.581 | 0.543 | 0.435 |

10/B3

| SNUC2B | 10 μM BI2852 | 20 μM BI2852 | 30 μM BI2852 |
|---|---|---|---|
| 1 nM TIPIFARNIB | 0.750 | 0.660 | 0.584 |
| 5 nM TIPIFARNIB | 0.700 | 0.624 | 0.610 |
| 25 nM TIPIFARNIB | 0.620 | 0.611 | 0.560 |
| 125 nM TIPIFARNIB | 0.520 | 0.530 | 0.493 |
| 625 nM TIPIFARNIB | 0.470 | 0.465 | 0.434 |

10/B4

| SW480 | 5 μM BI2852 | 10 μM BI2852 | 15 μM BI2852 |
|---|---|---|---|
| 100 nM TIPIFARNIB | 0.692 | 0.589 | 0.584 |
| 500 nM TIPIFARNIB | 0.653 | 0.499 | 0.496 |
| 1000 nM TIPIFARNIB | 0.539 | 0.451 | 0.436 |

10/B5

| A549 | 20 μM BI2852 | 30 μM BI2852 | 40 μM BI2852 |
|---|---|---|---|
| 25 nM TIPIFARNIB | 0.551 | 0.591 | 0.667 |
| 50 nM TIPIFARNIB | 0.682 | 0.711 | 0.740 |
| 100 nM TIPIFARNIB | 0.756 | 0.736 | 0.713 |

10/B6

| HCT116 | 10 μM BI2852 | 20 μM BI2852 | 30 μM BI2852 |
|---|---|---|---|
| 25 nM TIPIFARNIB | 0.657 | 0.710 | 0.769 |
| 50 nM TIPIFARNIB | 0.761 | 0.784 | 0.857 |
| 100 nM TIPIFARNIB | 0.579 | 0.684 | 0.634 |

10/B7

| SW480 | 15 μM BI2852 | 20 μM BI2852 | 30 μM BI2852 |
|---|---|---|---|
| 500 nM LONAFARNIB | 0.97012 | 0.91585 | 0.86151 |
| 1000 nM LONAFARNIB | 0.96358 | 0.84629 | 0.82327 |
| 2000 nM LONAFARNIB | 0.81662 | 0.75475 | 0.7354 |

Example 2: In vitro tumor cell proliferation assay using AMG510 and other prenylation inhibitors (statin, N-bisphosphonate, GGTI)

CI were also calculated with AMG510 and other prenylation inhibitors (statin: simvastatin (Table 12/A), N-bisphosphonate - BPH1222 (Table 12/B), GGTI - GGTI2418 (not applicable)) on two LUAD cells (H358, SW1573). CI values in SW1573 varied around 1 or higher, thus no synergism could be confirmed (Table 12). CI values in H358 at certain concentrations was below 1, thus mild synergism could be observed in this cell line with simvastatin and BPH1222 combination treatment with AMG510 (Table 12). In case of GGTI-2418 CI values cannot be calculated since GGTI-2418 was found to be ineffective at the concentration range of 1-20 μM (data not shown).

Tables 12 Combination indexes of AMG510 and simvastatin (A) and BPH1222 (B) combinational treatment. Combination indexes (CI) were calculated by CompuSyn Software from the data of viability assays (data not shown) of AMG510 and simvastatin or BPH1222 combination treatment. CI values less than 1 indicate synergy while values equal to or more than 1 represent additive or antagonistic effect, respectively.

11/A

| H358 | AMG-510 10 nM | AMG-510 25 nM | AMG-510 50 nM |
|---|---|---|---|
| SIMVASTATIN 1 μM | 1.22342 | 0.82769 | 0.71363 |
| SIMVASTATIN 2 μM | 1.20656 | 0.8578 | 0.70784 |
| SIMVASTATIN 4 μM | 0.89473 | 0.86923 | 0.54448 |
| SW1573 | AMG-510 10 nM | AMG-510 25 nM | AMG-510 50 nM |
| SIMVASTATIN 1 μM | 1.31129 | 1.33417 | 1.50149 |
| SIMVASTATIN 2 μM | 1.48451 | 1.6169 | 1.7549 |
| SIMVASTATIN 4 μM | 1.60939 | 1.70782 | 1.72535 |

11/B

| H358 | AMG-510 10 nM | AMG-510 25 nM | AMG-510 50 nM |
|---|---|---|---|
| BPH1222 1 μM | 0.76136 | 0.90437 | 0.8016 |
| BPH1222 2 μM | 0.66439 | 0.72068 | 0.73563 |
| BPH1222 4 μM | 0.54271 | 0.54587 | 0.69803 |
| SW1573 | AMG-510 10 nM | AMG-510 25 nM | AMG-510 50 nM |
| BPH1222 1 μM | 2.26654 | 1.27696 | 2.83465 |
| BPH1222 2 μM | 1.57979 | 1.17559 | 1.33918 |
| BPH1222 4 μM | 1.64125 | 1.47114 | 1.40638 |

11/A H358 viability data

| H358 | AMG-510 0 nM | | AMG-510 10 nM | | AMG-510 25 nM | | AMG-510 50 nM | | |
|---|---|---|---|---|---|---|---|---|---|
| | MEAN | SD | MEAN | SD | MEAN | SD | MEAN | SD | N |
| SIMVASTATIN 0 μM | 1.000 | 0.000 | 0.282 | 0.051 | 0.134 | 0.026 | 0.103 | 0.077 | 3 |
| SIMVASTATIN 1 μM | 0.914 | 0.034 | 0.266 | 0.030 | 0.123 | 0.003 | 0.064 | 0.011 | 3 |
| SIMVASTATIN 2 μM | 0.690 | 0.069 | 0.234 | 0.028 | 0.112 | 0.010 | 0.057 | 0.012 | 3 |
| SIMVASTATIN 4 μM | 0.388 | 0.163 | 0.132 | 0.066 | 0.088 | 0.020 | 0.034 | 0.003 | 3 |

11/A SW1573 viability data

| SW1573 | AMG-510 0 nM | | AMG-510 10 nM | | AMG-510 25 nM | | AMG-510 50 nM | | |
|---|---|---|---|---|---|---|---|---|---|
| | MEAN | SD | MEAN | SD | MEAN | SD | MEAN | SD | N |
| SIMVASTATIN 0 μM | 1.000 | 0.000 | 0.966 | 0.045 | 0.925 | 0.052 | 0.899 | 0.123 | 2 |
| SIMVASTATIN 1 μM | 0.670 | 0.088 | 0.758 | 0.079 | 0.743 | 0.120 | 0.748 | 0.145 | 2 |
| SIMVASTATIN 2 μM | 0.260 | 0.019 | 0.486 | 0.087 | 0.526 | 0.062 | 0.554 | 0.095 | 2 |
| SIMVASTATIN 4 μM | 0.077 | 0.010 | 0.191 | 0.055 | 0.212 | 0.029 | 0.214 | 0.047 | 2 |

11/B H358 viability data

| H358 | AMG-510 0 nM | | AMG-510 10 nM | | AMG-510 25 nM | | AMG-510 50 nM | | |
|---|---|---|---|---|---|---|---|---|---|
| | MEAN | SD | MEAN | SD | MEAN | SD | MEAN | SD | N |
| BPH1222 0 μM | 1.000 | 0.000 | 0.282 | 0.051 | 0.134 | 0.026 | 0.103 | 0.077 | 3 |
| BPH1222 1 μM | 0.914 | 0.034 | 0.266 | 0.030 | 0.123 | 0.003 | 0.064 | 0.011 | 3 |
| BPH1222 2 μM | 0.690 | 0.069 | 0.234 | 0.028 | 0.112 | 0.010 | 0.057 | 0.012 | 3 |
| BPH1222 4 μM | 0.388 | 0.163 | 0.132 | 0.066 | 0.088 | 0.020 | 0.034 | 0.003 | 3 |

11/B SW1573 viability data

| SW1573 | AMG-510 0 nM | | AMG-510 10 nM | | AMG-510 25 nM | | AMG-510 50 nM | | |
|---|---|---|---|---|---|---|---|---|---|
| | MEAN | SD | MEAN | SD | MEAN | SD | MEAN | SD | N |
| BPH1222 0 μM | 1.000 | | 0.995 | | 0.998 | | 0.977 | | 1 |
| BPH1222 1 μM | 0.802 | | 0.972 | | 0.863 | | 0.976 | | 1 |
| BPH1222 2 μM | 0.227 | | 0.621 | | 0.399 | | 0.490 | | 1 |
| BPH1222 4 μM | 0.056 | | 0.183 | | 0.139 | | 0.123 | | 1 |

Example 3: 3D culturing of tumor cells: effect of the combination of G12C - and FT inhibitors

Tables 12. Three-dimensional spheroid growth experiment was performed to assess the effect of AMG510 and tipifarnib combination treatment. Spheroid volume was detected by taking pictures during the treatment and at the end of the experiment. (A1-4) Spheres were treated with different concentrations of AMG510 or tipifarnib or both for six days. Relative cell viability based on tumor volume assessment on day 6 are shown (mean, SD, N). (B1-4) Combination indexes of AMG510 and tipifarnib combinational treatment. Combination indexes (CI) were calculated by CompuSyn Software from the data of viability assays of AMG510 and tipifarnib combination treatment. CI values less than 1 indicate synergy while values equal to or more than 1 represent additive or antagonistic effect, respectively.
12/A1

| H358 | 0 nM AMG510 | | 10 nM AMG510 | | 25 nM AMG510 | | 50 nM AMG510 | | |
|---|---|---|---|---|---|---|---|---|---|
| | MEAN | SD | MEAN | SD | MEAN | SD | MEAN | SD | N |
| 0 nM tipifarnib | 1 | 0 | 0.2936303 | 0.0235085 | 0.1441375 | 0.0187005 | 0.1009835 | 0.0107261 | 3 |
| 100 nM tipifarnib | 0.4856107 | 0.06006 | 0.1317135 | 0.026329 | 0.064059 | 0.0178293 | 0.0262721 | 0.005744 | 3 |
| 500 nM tipifarnib | 0.3484948 | 0.0093663 | 0.1071937 | 0.0169046 | 0.0438772 | 0.012386 | 0.0246174 | 0.0144067 | 3 |
| 1000 nM tipifarnib | 0.3289591 | 0.0097333 | 0.0842053 | 0.0151374 | 0.0306985 | 0.008523 | 0.0283188 | 0.0275539 | 3 |

12/A2

| H1792 | 0 nM AMG510 | | 10 nM AMG510 | | 25 nM AMG510 | | 50 nM AMG510 | | |
|---|---|---|---|---|---|---|---|---|---|
| | MEAN | SD | MEAN | SD | MEAN | SD | MEAN | SD | N |
| 0 nM tipifarnib | 1 | 0 | 0.8707951 | 0.1723098 | 0.8046703 | 0.1545024 | 0.6681424 | 0.1363197 | 3 |
| 100 nM tipifarnib | 0.4965543 | 0.1474645 | 0.4105126 | 0.1095683 | 0.4200511 | 0.111066 | 0.4126072 | 0.0922912 | 3 |
| 500 nM tipifarnib | 0.3852841 | 0.1076154 | 0.3284252 | 0.0676098 | 0.3187381 | 0.0590693 | 0.3251158 | 0.0965035 | 3 |
| 1000 nM tipifarnib | 0.3778354 | 0.1205365 | 0.343923 | 0.0697982 | 0.2748537 | 0.0827359 | 0.2774794 | 0.0419514 | 3 |

12/A3

| PF139 | 0 nM AMG510 | | 10 nM AMG510 | | 25 nM AMG510 | | 50 nM AMG510 | | |
|---|---|---|---|---|---|---|---|---|---|
| | MEAN | SD | MEAN | SD | MEAN | SD | MEAN | SD | N |
| 0 nM tipifarnib | 1 | 0 | 0.820712 | 0.0451045 | 0.7530033 | 0.0223306 | 0.7304567 | 0.0899457 | 3 |
| 100 nM tipifarnib | 0.7372998 | 0.0807743 | 0.6192425 | 0.0803313 | 0.4620792 | 0.1518028 | 0.3910501 | 0.0729747 | 3 |
| 500 nM tipifarnib | 0.5994549 | 0.0341666 | 0.4105941 | 0.0406621 | 0.3462551 | 0.0651571 | 0.3731988 | 0.0946093 | 3 |
| 1000 nM tipifarnib | 0.5518127 | 0.0662167 | 0.3021291 | 0.0841641 | 0.2944935 | 0.0571737 | 0.3371434 | 0.0652161 | 3 |

12/A4

| SW1573 | 0 nM AMG510 | | 10 nM AMG510 | | 25 nM AMG510 | | 50 nM AMG510 | | N |
|---|---|---|---|---|---|---|---|---|---|
| | MEAN | SD | MEAN | SD | MEAN | SD | MEAN | SD | |
| 0 nM tipifarnib | 1 | 0 | 0.8460324 | 0.010878 | 0.6947632 | 0.0918922 | 0.6026392 | 0.110969 | 3 |
| 100 nM tipifarnib | 0.7830464 | 0.0376718 | 0.5988367 | 0.062687 | 0.5051404 | 0.0351571 | 0.4210596 | 0.0469322 | 3 |
| 500 nM tipifarnib | 0.6774899 | 0.0897904 | 0.4702863 | 0.0690148 | 0.3456219 | 0.0519407 | 0.3117424 | 0.0310408 | 3 |
| 1000 nM tipifarnib | 0.5289958 | 0.0303714 | 0.3745545 | 0.0540661 | 0.2498178 | 0.0117614 | 0.2806062 | 0.0414103 | 3 |

12/B1

| H358 | AMG510 10 nM | AMG510 25 nM | AMG510 50 nM |
|---|---|---|---|
| 100 nM TIPIFARNIB | 0.313 | 0.295 | 0.188 |
| 500 nM TIPIFARNIB | 0.238 | 0.182 | 0.179 |
| 1000 nM TIPIFARNIB | 0.172 | 0.117 | 0.206 |

12/B2

| H1792 | AMG510 10 nM | AMG510 25 nM | AMG510 50 nM |
|---|---|---|---|
| 100 nM TIPIFARNIB | 0.277 | 0.389 | 0.461 |
| 500 nM TIPIFARNIB | 0.263 | 0.260 | 0.354 |
| 1000 nM TIPIFARNIB | 0.681 | 0.201 | 0.257 |

12/B3

| PF139 | AMG510 10 nM | AMG510 25 nM | AMG510 50 nM |
|---|---|---|---|
| 100 nM TIPIFARNIB | 0.278 | 0.058 | 0.033 |
| 500 nM TIPIFARNIB | 0.115 | 0.056 | 0.084 |
| 1000 nM TIPIFARNIB | 0.061 | 0.056 | 0.101 |

12/B4

| SW1573 | AMG510 10 nM | AMG510 25 nM | AMG510 50 nM |
|---|---|---|---|
| 100 nM TIPIFARNIB | 0.351 | 0.391 | 0.461 |
| 500 nM TIPIFARNIB | 0.347 | 0.225 | 0.298 |
| 1000 nM TIPIFARNIB | 0.276 | 0.143 | 0.284 |

3D growth inhibition assays were also performed with single agents or a combination of AMG510 and tipifarnib on our panel of KRAS G12C mutant LUAD cell lines to further confirm synergism. Drug sensitivity to AMG510 and Tipifarnib was similar to 2D effects in most of the cell lines, however SW1573 showed higher sensitivity to AMG510 in 3D compared to 2D settings (Table 5A). Also, CI values represented strong synergism similar to 2D results.

In conclusion, general synergistic (all CI values are below 1) combinational effects with KRAS G12C inhibitors could only be observed using farnesyl-transferase inhibitors. However, synergistic effects of this combination have been shown to be highly robust, as it was independent of tissue of origins and could be observed in all cell lines even if we combined other drugs of these drug classes.

Example 4: Combinational treatments of human lung adenocarcinoma *in vivo* in SCID mice

We examined the effect of AMG510, tipifarnib or combined treatment on *in vivo* growth of H358 and SW1573 cells transplanted subcutaneously into female SCID mice (Figure 2-3). Notably, H358 was showed to be highly sensitive to AMG510 treatment, while SW1573 was resistant in 2D *in vitro* experiments. Thus, AMG510 dosage differed accordingly.

Once tumors reached approx. 100 mm$^3$ volume animals received either vehicle as control or AMG510 at 5 mg (H358) / 25 mg (SW1573) or tipifarnib at 40 mg or both (per kg body weight) daily, except for weekends by intraperitoneal injection. Tipifarnib monotherapy resulted in strong tumor inhibition in the SW1573 tumor carrying homozygous mutant KRAS, however AMG510 monotherapy could effectively inhibited tumors of both cell lines (Figure 2-3).

More importantly, combinational treatment resulted in the most pronounced inhibition of tumor growth, as demonstrated by either the tumor volume values measured during the experiment and the tumor weight values that was measured after termination (Figure 2,3). Of note, based on tumor weight, only combinational treatment was able to block tumor growth in H358 xenografts compared to control (Figure 2/B). To assess the toxicity associated with the drug treatment, body weights were monitored throughout the course of the study. Body weight losses were not significantly different between treatment groups (Figure 2/C, Figure 3/C).

Table 13. Weights of the tumors at the end of the experiment upon treatment of mice carrying H358 xenografts with AMG510 (5 mg/kg i.p.) and/or tipifarnib (40 mg/kg i.p.) (day1=1).

|  | CONTROL | AMG510 | TIPIFARNIB | AMG+TIPI |
|---|---|---|---|---|
| 1 | 0.466 | 0.053 | 0.182 | 0.144 |
| 2 | 0.612 | 0.559 | 1.599 | 0.038 |
| 3 | 0.242 | 0.038 | 1.023 | 0.027 |
| 4 | 0.403 | 0.157 | 0.223 | 0.021 |
| 5 | 0.445 | n.a. | 0.225 | 0.029 |
| 6 | 0.459 | 0.254 | 0.205 | 0.036 |
| 7 | 0.389 | 0.029 | 0.239 | 0.234 |
| MEAN | 0.4308571 | 0.1816667 | 0.528 | 0.0755714 |
| SEM | 0.04176 | 0.08341 | 0.2118 | 0.03096 |
| N | 7 | 6 | 7 | 7 |

Table 14. Weights of the tumors at the end of the experiment upon treatment of mice carrying of SW1573 xenografts with AMG510 (25 mg/kg i.p.) and/or tipifarnib (40 mg/kg i.p.) (day1=1).

|  | CONTROL | AMG510 | TIPIFARNIB | AMG+TIPI |
|---|---|---|---|---|
| 1 | 0.351 | 0.1025 | 0.5323 | 0.0318 |
| 2 | 0.76 | n.a. | 0.2277 | 0.4871 |
| 3 | n.a | 0.7238 | 0.3143 | 0.4632 |
| 4 | 0.6163 | 0.2227 | n.a | 0.266 |
| 5 | 1.377 | 0.144 | 0.2239 | 0.1962 |
| 6 | 0.7105 | 0.5804 | 0.2604 | 0.1802 |
| 7 | 0.1855 | 0.4184 | 1.2243 | 0.0455 |
| MEAN | 0.666717 | 0.3653 | 0.463817 | 0.238571 |
| SEM | 0.1682 | 0.1026 | 0.1592 | 0.0687 |
| N | 6 | 6 | 6 | 7 |

In conclusion it can be stated that while heterozygous G12C mutant human cancer cell line (H358) was more sensitive to G12C inhibitor than the homozygous KRAS mutant cell line (SW1573), the combination of G12C inhibitor with FTI resulted in the most effective and robust *in vivo* antitumoral effect.

Example 5: The role of HRAS in the mechanism of action of synergistic antitumoral effects of combinational therapy

Investigation of protein farnesylation and RAS activation was performed using SW1573 cell lines. This cell line was chosen for detailed investigation because it harbors homozygous KRAS G12C mutation, therefore all changes in KRAS4B represent only the mutant oncoprotein. Western blot analyses revealed that inhibition of farnesyl-transferase by tipifarnib did not interfere with KRAS4B and NRAS, while it effectively blocked farnesylation of HRAS proteins both in mono- and combinational therapy, where a small, but clear shift in electrophoretic mobility could be observed (upper band represents unfarnesylated proteins) (Figure 6a). In line with these results, GTP-KRAS4B was only decreased upon sotorasib and combinational treatment. GTP-NRAS were at low detection level and apparently did not changed upon treatment. Interestingly, level of GTP-HRAS increased upon sotorasib treatment compensating loss of active KRAS4B. Furthermore, GTP-HRAS upon tipifarnib and combinational treatment was mainly found at the non-prenylated fraction, that is not able to contribute properly to RAS signalling [17]. Thus, combinational treatment resulted in decreased functioning active GTP-KRAS4B and GTP-HRAS.

To further test role of HRAS in mechanism of action of synergistic antitumoral effects of combinational therapy, we knocked out HRAS with siRNA and determined changes in IC50 values of sotorasib and tipifarnib (Figure 6c). We found that upon siRNA knockdown of HRAS, IC50 of sotorasib decreased from 99.98 nM to 51.74 nM, while IC50 of tipifarnib increased from 4.561 nM to 10.47 nM.

Table 15. Changes of IC50 values upon transfection with non-targeting and HRAS-targeting siRNA upon 6-day-long treatment with tipifarnib and sotorasib. All data shown derives from three independent experiments.

| SW1573 | Cont siRNA | HRAS siRNA |
|---|---|---|
| Tipifarnib IC50 (nM) | 4.561 | 10.47 |
| Sotorasib IC50 (nM) | 99.98 | 51.74 |

**References**

1. Buday, L. and J. Downward, Many faces of Ras activation. Biochim Biophys Acta, 2008. 1786(2): p. 178-87.

2. Cox, A.D. and C.J. Der, Ras history: The saga continues. Small GTPases, 2010. 1(1): p. 2-27.

3. Simanshu, D.K., D.V. Nissley, and F. McCormick, RAS Proteins and Their Regulators in Human Disease. Cell, 2017. 170(1): p. 17-33.

4. Janes, M.R., et al., Targeting KRAS Mutant Cancers with a Covalent G12C-Specific Inhibitor. Cell, 2018. 172(3): p. 578-+.

5. Hong, D.S., et al., KRAS(G12C) Inhibition with Sotorasib in Advanced Solid Tumors. N Engl J Med, 2020. 383(13): p. 1207-1217.

6. Hallin, J., et al., The KRAS(G12C) Inhibitor MRTX849 Provides Insight toward Therapeutic Susceptibility of KRAS-Mutant Cancers in Mouse Models and Patients. Cancer Discov, 2020. 10(1): p. 54-71.

7. Moore, A.R., et al., RAS-targeted therapies: is the undruggable drugged? Nat Rev Drug Discov, 2020. 19: p. 533-552.

8. Dunnett-Kane, V., et al., Mechanisms of Resistance to KRAS(G12C) Inhibitors. Cancers (Basel), 2021. 13(1).

9. Macdonald, J.S., et al., A phase II study of farnesyl transferase inhibitor R115777 in pancreatic cancer: a Southwest oncology group (SWOG 9924) study. Invest New Drugs, 2005. 23(5): p. 485-7.

10. Tsimberidou, A.M., et al., Phase 1 first-in-human clinical study of S-trans,trans-farnesylthiosalicylic acid (salirasib) in patients with solid tumors. Cancer Chemother Pharmacol, 2010. 65(2): p. 235-41.

11. Laheru, D., et al., Integrated preclinical and clinical development of S-trans, trans-Farnesylthiosalicylic Acid (FTS, Salirasib) in pancreatic cancer. Invest New Drugs, 2012. 30(6): p. 2391-9.

12. Baranyi, M., et al., Next Generation Lipophilic Bisphosphonate Shows Antitumor Effect in Colorectal Cancer In Vitro and In Vivo. Pathol Oncol Res, 2020. 26: p. 1957-1969.

13. Ivanov, D.P., et al., Multiplexing spheroid volume, resazurin and acid phosphatase viability assays for high-throughput screening of tumour spheroids and stem cell neurospheres. PLoS One, 2014. 9(8): p. e103817.

14. Whyte D. B., et al., K- and N-Ras are geranylgeranylated in cells treated with farnesyl protein transferase inhibitors. J Biol Chem. 1997 May 30;272(22):14459-64.

15. van Cutsem E, van de Velde H, Karasek P, et al. Phase III trial of gemcitabine plus tipifarnib compared with gemcitabine plus placebo in advanced pancreatic cancer. J Clin Oncol. 2004;22(8):1430-1438.

16. Ghimessy, A., Radeczky, P., Laszlo, V. et al. Current therapy of KRAS-mutant lung cancer. Cancer Metastasis Rev 39, 1159-1177 (2020).

17. Christensen, J. G., et al., Targeting Krasg12c-mutant cancer with a mutation-specific inhibitor. JIM https://doi.org/10.1111/joim.13057, 2020

18. Lindsay, C. R. et al., KRAS: Reasons for optimism in lung cancer. EJC Volume 99, P20-27, August 01, 2018

**Claims**

**1.** Farnesyl transferase inhibitor (FTI) compound for use in the treatment of a KRAS mutation carrying cancer,

wherein the FTI compound is used in combination with a KRAS inhibitor compound
wherein the FTI compound is selected from tipifarnib or lonafarnib and

the KRAS inhibitor compound is selected from sotorasib, adagrasib, MRTX1133, BI2852.

2. The farnesyl transferase inhibitor (FTI) compound for use according to claim 1,

wherein KRAS mutation is G12C,
the KRAS inhibitor compound is sotorasib or adagrasib.

3. The farnesyl transferase inhibitor (FTI) compound for use according to claim 1,

wherein KRAS mutation is G12D,
the KRAS inhibitor compound is MRTX1133.

4. The farnesyl transferase inhibitor (FTI) compound for use according to claim 1,

wherein KRAS mutation is G12C,
the KRAS inhibitor compound is adagrasib.

5. The farnesyl transferase inhibitor (FTI) compound for use according to claim 4, wherein the cancer is selected from lung, colorectal and pancreatic cancer.

6. The farnesyl transferase inhibitor (FTI) compound for use according to claim 1,

wherein KRAS mutation is G12C,
the FTI compound is tipifarnib, the KRAS inhibitor compound is sotorasib and
the cancer is selected from lung, colorectal and pancreatic cancer.

7. The farnesyl transferase inhibitor (FTI) compound for use according to claim 3,
the FTI compound is tipifarnib and the cancer is selected from lung, colorectal and pancreatic cancer.

8. The farnesyl transferase inhibitor (FTI) compound for use according to claim 3,
the FTI compound is lonafarnib and the cancer is pancreatic cancer.

9. The farnesyl transferase inhibitor (FTI) compound for use according to claim 1,

wherein the KRAS mutation is G12C,
the FTI compound is tipifarnib and the KRAS inhibitor compound is BI2852.

10. The farnesyl transferase inhibitor (FTI) compound for use according to claim 9, wherein the cancer is lung cancer or colorectal cancer.

11. The farnesyl transferase inhibitor (FTI) compound for use according to claim 1,

wherein the KRAS mutation is G12D,
the FTI compound is tipifarnib and the KRAS inhibitor compound is BI2852.

12. The farnesyl transferase inhibitor (FTI) compound for use according to claim 11, wherein the cancer is pancreatic or colorectal cancer.

13. Farnesyl transferase inhibitor (FTI) compound for use in the treatment of a KRAS mutation carrying cancer, wherein the FTI compound is used in combination with a KRAS inhibitor compound wherein the FTI compound is tipifarnib and the KRAS inhibitor compound is ARS1620, preferably wherein the cancer is lung cancer.

14. Farnesyl transferase inhibitor (FTI) compound for use in the treatment of a KRAS mutation carrying cancer, wherein the FTI compound is used in combination with a KRAS inhibitor compound wherein the FTI compound is FTI277 or BMS214662 and the KRAS inhibitor compound is sotorasib, preferably wherein the cancer is lung cancer.

15. The farnesyl transferase inhibitor (FTI) compound for use according to any one of the preceding claims, wherein the FTI compound and the KRAS inhibitor compound are administered to a patient having the KRAS mutation carrying cancer, in amounts effective to induce a synergistic effect on the cancer cells.

**Patentansprüche**

1.  Farnesyltransferase-Inhibitor-Verbindung (FTI-Verbindung) zur Verwendung bei der Behandlung von einem Krebs mit eine KRAS-Mutation, wobei die FTI-Verbindung in Kombination mit einer KRAS-Inhibitor-Verbindung verwendet wird,

    wobei die FTI-Verbindung aus Tipifarnib und Lonafarnib ausgewählt ist und
    die KRAS-Inhibitor-Verbindung aus Sotorasib, Adagrasib, MRTX1133 und BI2852 ausgewählt ist.

2.  Die Farnesyltransferase-Inhibitor-Verbindung (FTI-Verbindung) zur Verwendung nach Anspruch 1, wobei die KRAS-Mutation G12C ist,
    die KRAS-Inhibitor-Verbindung Sotorasib oder Adagrasib ist.

3.  Die Farnesyltransferase-Inhibitor-Verbindung (FTI-Verbindung) zur Verwendung nach Anspruch 1, wobei die KRAS-Mutation G12D ist,
    die KRAS-Inhibitor-Verbindung MRTX1133 ist.

4.  Die Farnesyltransferase-Inhibitor-Verbindung (FTI-Verbindung) zur Verwendung nach Anspruch 1, wobei die KRAS-Mutation G12C ist,
    die KRAS-Inhibitor-Verbindung ist.

5.  Die Farnesyltransferase-Inhibitor-Verbindung (FTI-Verbindung) zur Verwendung nach Anspruch 4, wobei der Krebs aus Lungenkrebs, Kolorektalkrebs und Bauchspeicheldrüsenkrebs ausgewählt ist.

6.  Die Farnesyltransferase-Inhibitor-Verbindung (FTI-Verbindung) zur Verwendung nach Anspruch 1, wobei die KRAS-Mutation G12C ist,

    die FTI-Verbindung Tipifarnib ist, die KRAS-Inhibitor-Verbindung Sotorasib ist und
    der Krebs aus Lungenkrebs, Kolorektalkrebs und Bauchspeicheldrüsenkrebs ausgewählt ist.

7.  Die Farnesyltransferase-Inhibitor-Verbindung (FTI-Verbindung) zur Verwendung nach Anspruch 3, wobei die FTI-Verbindung Tipifarnib ist und der Krebs aus Lungenkrebs, Kolorektalkrebs und Bauchspeicheldrüsenkrebs ausgewählt ist.

8.  Die Farnesyltransferase-Inhibitor-Verbindung (FTI-Verbindung) zur Verwendung nach Anspruch 3, wobei die FTI-Verbindung Tipifarnib ist und der Krebs Bauchspeicheldrüsenkrebs ist.

9.  Die Farnesyltransferase-Inhibitor-Verbindung (FTI-Verbindung) zur Verwendung nach Anspruch 1, wobei die KRAS-Mutation G12C ist,
    die FTI-Verbindung Tipifarnib ist und die KRAS-Inhibitor-Verbindung BI2852 ist.

10. Die Farnesyltransferase-Inhibitor-Verbindung (FTI-Verbindung) zur Verwendung nach Anspruch 9, wobei es sich bei dem Krebs um Lungenkrebs oder Kolorektalkrebs handelt.

11. Die Farnesyltransferase-Inhibitor-Verbindung (FTI-Verbindung) zur Verwendung nach Anspruch 1, wobei die KRAS-Mutation G12D ist,
    die FTI-Verbindung Tipifarnib ist und die KRAS-Inhibitor-Verbindung BI2852 ist.

12. Die Farnesyltransferase-Inhibitor-Verbindung (FTI-Verbindung) zur Verwendung nach Anspruch 11, wobei es sich bei der Krebserkrankung um Bauchspeicheldrüsenkrebs oder Kolorektalkrebs handelt.

13. Farnesyltransferase-Inhibitor-Verbindung (FTI-Verbindung) zur Verwendung bei der Behandlung von einem Krebs mit eine KRAS-Mutation, wobei die FTI-Verbindung in Kombination mit einer KRAS-Inhibitor-Verbindung verwendet wird,
    wobei die FTI-Verbindung Tipifarnib ist und die KRAS-Inhibitor-Verbindung ARS1620 ist, vorzugsweise wobei der Krebs Lungenkrebs ist.

14. Farnesyltransferase-Inhibitor-Verbindung (FTI-Verbindung) zur Verwendung bei der Behandlung von einem Krebs mit eine KRAS-Mutation, wobei die FTI-Verbindung in Kombination mit einer KRAS-Inhibitor-Verbindung verwendet wird, wobei die FTI-Verbindung FTI277 oder BMS214662 ist und die KRAS-Inhibitor-Verbindung Sotorasib ist, vorzugsweise wobei der Krebs Lungenkrebs ist.

15. Die Farnesyltransferase-Inhibitor-Verbindung (FTI-Verbindung) zur Verwendung nach einem der vorhergehenden Ansprüche, wobei die FTI-Verbindung und die KRAS-Inhibitor-Verbindung einem Patienten mit einem Krebs mit einer KRAS-Mutation in Mengen verabreicht werden, die eine synergistische Wirkung auf die Krebszellen hervorrufen.

**Revendications**

1. Composé inhibiteur de la farnésyl transférase (FTI) pour une utilisation dans le traitement d'un cancer présentant une mutation de KRAS, le composé FTI étant utilisé en association avec un composé inhibiteur de KRAS,

le composé FTI étant choisi parmi le tipifarnib ou le lonafarnib, et
le composé inhibiteur de KRAS étant choisi parmi le sotorasib, l'adagrasib, le MRTX1133 et le BI2852.

2. Composé inhibiteur de la farnésyl transférase (FTI) pour une utilisation selon la revendication 1,

la mutation de KRAS étant G12C,
le composé inhibiteur de KRAS étant le sotorasib ou l'adagrasib.

3. Composé inhibiteur de la farnésyl transférase (FTI) pour une utilisation selon la revendication 1,

la mutation de KRAS étant G12D,
le composé inhibiteur de KRAS étant le MRTX1133.

4. Composé inhibiteur de la farnésyl transférase (FTI) pour une utilisation selon la revendication 1,

la mutation de KRAS étant G12C,
le composé inhibiteur de KRAS étant l'adagrasib.

5. Composé inhibiteur de la farnésyl transférase (FTI) pour une utilisation selon la revendication 4, le cancer étant choisi parmi les cancers du poumon, colorectal et du pancréas.

6. Composé inhibiteur de la farnésyl transférase (FTI) pour une utilisation selon la revendication 1,

la mutation de KRAS étant G12C,
le composé FTI étant le tipifarnib, le composé inhibiteur de KRAS étant le sotorasib, et
le cancer étant choisi parmi les cancers du poumon, colorectal et du pancréas.

7. Composé inhibiteur de la farnésyl transférase (FTI) pour une utilisation selon la revendication 3, le composé FTI étant le tipifarnib, et le cancer étant choisi parmi les cancers du poumon, colorectal et du pancréas.

8. Composé inhibiteur de la farnésyl transférase (FTI) pour une utilisation selon la revendication 3, le composé FTI étant le lonafarnib et le cancer étant le cancer du pancréas.

9. Composé inhibiteur de la farnésyl transférase (FTI) pour une utilisation selon la revendication 1,

la mutation de KRAS étant G12C,
le composé FTI étant le tipifarnib et le composé inhibiteur de KRAS étant le BI2852.

10. Composé inhibiteur de la farnésyl transférase (FTI) pour une utilisation selon la revendication 9, le cancer étant le cancer du poumon ou le cancer colorectal.

**11.** Composé inhibiteur de la farnésyl transférase (FTI) pour une utilisation selon la revendication 1,

> la mutation de KRAS étant G12D,
> le composé FTI étant le tipifarnib et le composé inhibiteur de KRAS étant le BI2852.

**12.** Composé inhibiteur de la farnésyl transférase (FTI) pour une utilisation selon la revendication 11, le cancer étant le cancer du pancréas ou le cancer colorectal.

**13.** Composé inhibiteur de la farnésyl transférase (FTI) pour une utilisation dans le traitement d'un cancer présentant une mutation de KRAS, le composé FTI étant utilisé en association avec un composé inhibiteur de KRAS,
le composé FTI étant le tipifarnib, et le composé inhibiteur de KRAS étant l'ARS1620, le cancer étant de préférence le cancer du poumon.

**14.** Composé inhibiteur de la farnésyl transférase (FTI) pour une utilisation dans le traitement d'un cancer présentant une mutation de KRAS, le composé FTI étant utilisé en association avec un composé inhibiteur de KRAS,
le composé FTI étant le FTI277 ou le BMS214662, et le composé inhibiteur de KRAS étant le sotorasib, le cancer étant de préférence le cancer du poumon.

**15.** Composé inhibiteur de farnésyl transférase (FTI) pour une utilisation selon l'une quelconque des revendications précédentes, le composé FTI et le composé inhibiteur de KRAS étant administrés à un patient atteint du cancer présentant une mutation de KRAS, dans des quantités efficaces pour induire un effet synergique sur les cellules cancéreuses.

**FIGURE 1**

# H358

**FIGURE 2A**

# H358

**FIGURE 2B**

# H358

**FIGURE 2C**

**FIGURE 2D**

**FIGURE 3A**

## SW1573

**FIGURE 3B**

## SW1573

**FIGURE 3C**

FIGURE 3D

FIGURE 4A

**FIGURE 4B**

# EP 4 426 303 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1165078 A
- WO 2021097207 A

### Non-patent literature cited in the description

- Proceedings of the 112th Annual Meeting. American Association for Cancer Research, 17 May 2021
- **MAO, Z ; XIAO, H. ; SHEN, P et al.** KRASG12D can be targeted by potent inhibitors via formation of salt bridge.. *Cell Discov*, 2022, vol. 8, 5, https://doi.org/10.1038
- **HOFMAN MH et al.** Expanding the reach of precision oncology by drugging all KRAS mutants.. *Cancer Discovery*, 2022, vol. 12, 924
- **SKOULIDIS F ; LI BT ; DY GK ; PRICE TJ ; FALCHOOK GS ; WOLF J et al.** Sotorasib for lung cancers with KRAS p.G12C mutation.. *N Engl J Med*, 2021, vol. 384, 2371-81
- **A.A. ADJE et al.** Phase II study of the farnesyl transferase inhibitor R115777 in patients with advanced non-small-cell lung cancer.. *J Clin Oncol*, 2003, vol. 21 (9), 1760-1766
- **J.V. HEYMACH et al.** Phase II study of the farnesyl transferase inhibitor R115777 in patients with sensitive relapse small-cell lung cancer.. *Ann Oncol*, 2004, vol. 15 (8), 1187-1193
- **C. YAM et al.** A phase II study of tipifarnib and gemcitabine in metastatic breast cancer.. *Invest New Drugs*, 2018, vol. 36 (2), 299-306
- **S. SHARMA et al.** A phase II trial of farnesyl protein transferase inhibitor SCH 66336, given by twice-daily oral administration, in patients with metastatic colorectal cancer refractory to 5-fluorouracil and irinotecan.. *Ann Oncol*, 2002, vol. 13 (7), 1067-1071
- **W. MEIER et al.** Randomized phase II trial of carboplatin and paclitaxel with or without lonafarnib in first-line treatment of epithelial ovarian cancer stage IIB-IV.. *Gynecol Oncol*, 2012, vol. 126 (2), 236-240
- **GHIMESSY, A. et al.** Current therapy of KRAS-mutant lung cancer.. *Cancer Metastasis Rev*, 2020, vol. 39, 1159-1177
- KRAS Inhibitors- yes but what next? Direct targeting of KRAS- vaccines, adoptive T cell therapy and beyond. **NAGASAKA MISAKO et al.** CANCER TREATMENT REVIEWS. ELSEVIER, 21 October 2021, vol. 101
- **KUMAR, V ; ABBAS, A. ; ASTER, J.** Basic pathology. Elsevier, 2017
- **BUDAY, L ; J. DOWNWARD**. Many faces of Ras activation.. *Biochim Biophys Acta*, 2008, vol. 1786 (2), 178-87
- **COX, A.D. ; C.J. DER**. Ras history: The saga continues.. *Small GTPases*, 2010, vol. 1 (1), 2-27
- **SIMANSHU, D.K ; D.V. NISSLEY ; F. MCCORMICK**. RAS Proteins and Their Regulators in Human Disease.. *Cell*, 2017, vol. 170 (1), 17-33
- **JANES, M.R et al.** Targeting KRAS Mutant Cancers with a Covalent G12C-Specific Inhibitor.. *Cell*, 2018, vol. 172 (3), 578
- **HONG, D.S. et al.** KRAS(G12C) Inhibition with Sotorasib in Advanced Solid Tumors.. *N Engl J Med*, 2020, vol. 383 (13), 1207-1217
- **HALLIN, J et al.** The KRAS(G12C) Inhibitor MRTX849 Provides Insight toward Therapeutic Susceptibility of KRAS-Mutant Cancers in Mouse Models and Patients.. *Cancer Discov*, 2020, vol. 10 (1), 54-71
- **MOORE, A.R. et al.** RAS-targeted therapies: is the undruggable drugged. *Nat Rev Drug Discov*, 2020, vol. 19, 533-552
- **DUNNETT-KANE, V. et al.** Mechanisms of Resistance to KRAS(G12C) Inhibitors.. *Cancers*, 2021, vol. 13 (1)
- **MACDONALD, J.S. et al.** A phase II study of farnesyl transferase inhibitor R115777 in pancreatic cancer: a Southwest oncology group (SWOG 9924) study.. *Invest New Drugs*, 2005, vol. 23 (5), 485-7
- **TSIMBERIDOU, A.M. et al.** Phase 1 first-in-human clinical study of S-trans,trans-farnesylthiosalicylic acid (salirasib) in patients with solid tumors.. *Cancer Chemother Pharmacol*, 2010, vol. 65 (2), 235-41
- **LAHERU, D. et al.** Integrated preclinical and clinical development of S-trans, trans-Farnesylthiosalicylic Acid (FTS, Salirasib) in pancreatic cancer.. *Invest New Drugs*, 2012, vol. 30 (6), 2391-9

- **BARANYI, M. et al.** Next Generation Lipophilic Bisphosphonate Shows Antitumor Effect in Colorectal Cancer In Vitro and In Vivo.. *Pathol Oncol Res*, 2020, vol. 26, 1957-1969
- **IVANOV, D.P. et al.** Multiplexing spheroid volume, resazurin and acid phosphatase viability assays for high-throughput screening of tumour spheroids and stem cell neurospheres.. *PLoS One*, 2014, vol. 9 (8), e103817
- **WHYTE D. B. et al.** K- and N-Ras are geranylgeranylated in cells treated with farnesyl protein transferase inhibitors.. *J Biol Chem.*, 30 May 1997, vol. 272 (22), 14459-64
- **VAN CUTSEM E** ; **VAN DE VELDE H** ; **KARASEK P et al.** Phase III trial of gemcitabine plus tipifarnib compared with gemcitabine plus placebo in advanced pancreatic cancer.. *J Clin Oncol.*, 2004, vol. 22 (8), 1430-1438
- **GHIMESSY, A** ; **RADECZKY, P.** ; **LASZLO, V. et al.** Current therapy of KRAS-mutant lung cancer. *Cancer Metastasis Rev*, 2020, vol. 39, 1159-1177
- **CHRISTENSEN, J. G. et al.** Targeting Krasg12c-mutant cancer with a mutation-specific inhibitor.. *JIM*, 2020, https://doi.org/10.1111/joim.13057
- **LINDSAY, C. R. et al.** KRAS: Reasons for optimism in lung cancer.. *EJC*, 01 August 2018, vol. 99, 20-27